(19) Europäisches Patentamt
European Patent Office
Office européen des brevets

(11) EP 4 486 097 A1

(12) **EUROPEAN PATENT APPLICATION**
published in accordance with Art. 153(4) EPC

(43) Date of publication:
**01.01.2025 Bulletin 2025/01**

(21) Application number: **23759705.9**

(22) Date of filing: **09.02.2023**

(51) International Patent Classification (IPC):
***H10K 85/60*** *(2023.01)* ***C07D 209/80*** *(2006.01)*
***C09K 11/06*** *(2006.01)* ***H10K 50/12*** *(2023.01)*
***H10K 71/16*** *(2023.01)* ***H10K 71/30*** *(2023.01)*
***H10K 101/10*** *(2023.01)* ***H10K 101/20*** *(2023.01)*

(52) Cooperative Patent Classification (CPC):
**C07D 209/80; C09K 11/06; H10K 50/12; H10K 71/16; H10K 71/30; H10K 85/60;**
H10K 2101/10; H10K 2101/20

(86) International application number:
**PCT/JP2023/004387**

(87) International publication number:
**WO 2023/162701 (31.08.2023 Gazette 2023/35)**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(30) Priority: **25.02.2022 JP 2022028707**

(71) Applicant: **NIPPON STEEL Chemical & Material Co., Ltd.**
**Tokyo 103-0027 (JP)**

(72) Inventors:
- **TADA, Masashi**
  **Tokyo 103-0027 (JP)**
- **OGAWA, Junya**
  **Tokyo 103-0027 (JP)**
- **TERADA, Ayaka**
  **Tokyo 103-0027 (JP)**

(74) Representative: **Kraus & Lederer PartGmbB**
**Thomas-Wimmer-Ring 15**
**80539 München (DE)**

(54) **MATERIAL FOR ORGANIC ELECTROLUMINESCENT ELEMENT, AND ORGANIC ELECTROLUMINESCENT ELEMENT**

(57) Provided is an organic electroluminescent device having a low driving voltage, high emission efficiency, and a long lifetime. In an organic electroluminescent device including one or more organic layers between an anode and a cathode opposite to each other, at least one of the organic layers contains a material for an organic electroluminescent device represented by the following general formula (1), so that an organic electroluminescent device having a low driving voltage, high emission efficiency, and a long lifetime is provided.

EP 4 486 097 A1

[C1]

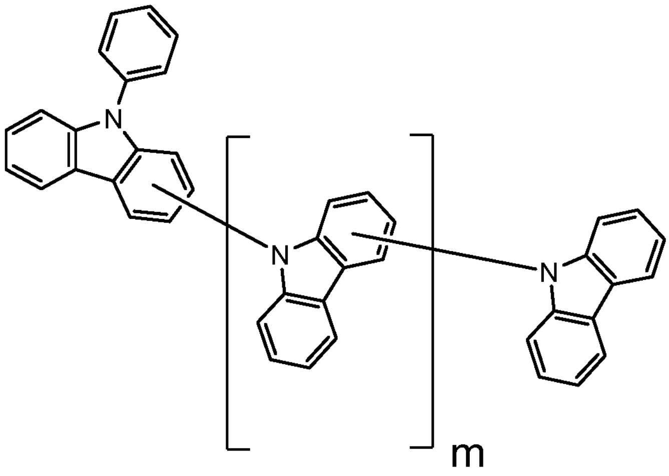

(1)

[FIG. 1]

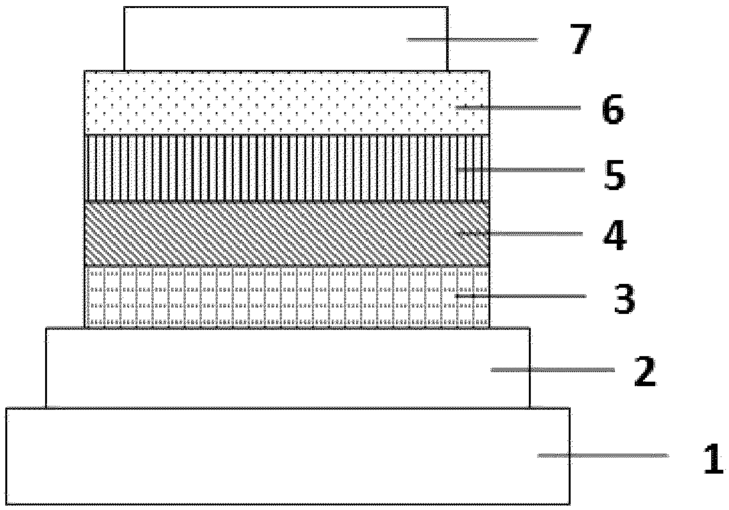

## Description

### Technical Field

The present invention relates to an organic electroluminescent device (also referred to as an organic EL device) that can transform electric energy into light, and a material for an organic electroluminescent device used therein.

When a voltage is applied to an organic EL device, holes and electrons are injected from the anode and the cathode, respectively, into the light emitting layer. Then, the injected holes and electrons are recombined in the light emitting layer to thereby generate excitons. At this time, according to the electron spin statistics theory, singlet excitons and triplet excitons are generated at a ratio of 1:3. In the fluorescent organic EL device that uses emission caused by singlet excitons, the limit of the internal quantum efficiency is said to be 25%. On the other hand, it has been known that, in the phosphorescent organic EL device that uses emission caused by triplet excitons, the internal quantum efficiency can be enhanced up to 100% when intersystem crossing efficiently occurs from singlet excitons.

A technology for extending the lifetime of a phosphorescent organic EL device has advanced in recent years, and the device is being applied to a display of a mobile phone and others. Regarding a blue organic EL device, however, a practical phosphorescent organic EL device has not been developed, and thus the development of a blue organic EL device having high efficiency and a long lifetime is desired.

Further, a highly efficient delayed fluorescence organic EL device utilizing delayed fluorescence has been developed, in recent years. For example, Patent Literature 1 discloses an organic EL device utilizing the Triplet-Triplet Fusion (TTF) mechanism, which is one of the mechanisms of delayed fluorescence. The TTF mechanism utilizes a phenomenon in which a singlet exciton is generated by the collision of two triplet excitons, and it is believed that the internal quantum efficiency can be enhanced up to 40%, in theory. However, its efficiency is low as compared with the efficiency of the phosphorescent organic EL device, and thus further improvement in efficiency is desired.

On the other hand, Patent Literature 2 discloses an organic EL device utilizing the Thermally Activated Delayed Fluorescence (TADF) mechanism. The TADF mechanism utilizes a phenomenon in which reverse intersystem crossing occurs from the triplet exciton to the singlet exciton in a material having a small energy difference between the singlet level and the triplet level, and it is believed that the internal quantum efficiency can be enhanced up to 100%, in theory.

The driving voltage, emission efficiency, and lifetime characteristics of the organic EL device largely depend on electron transporting materials that transport charges such as holes and electrons to the light emitting layer and host materials in the light emitting layer. Among them, materials having a carbazole backbone are known as the host material of the light emitting layer (e.g., see Patent Literatures 3 to 6).

Also, it is known that when a material having a carbazole backbone is used as the host material of the light emitting layer, a deuterated host material is used (e.g., see Patent Literatures 7 to 8).

### Citation List

#### Patent Literature

Patent Literature 1: WO2010/134350 A
Patent Literature 2: WO2011/070963 A
Patent Literature 3: WO2012/077520 A
Patent Literature 4: WO2016/158191 A
Patent Literature 5: WO2018/173593 A
Patent Literature 6: WO2021/200252 A
Patent Literature 7: US2022/0020939
Patent Literature 8: US2022/0029106

### Summary of Invention

#### Technical Problem

In view of applying an organic EL device to a display device such as a flat panel display and a light source, it is necessary to improve the emission efficiency of the device and sufficiently ensure the stability of the device at the time of driving, at the same time. The present invention has been made under such circumstances, and an object thereof is to provide a material for an organic electroluminescent device that has a low driving voltage, high emission efficiency, and long lifetime characteristics, and allows a practically useful organic EL device to be obtained, and an organic EL device

including the material for an organic electroluminescent device.

Solution to Problem

The present invention is a material for an organic electroluminescent device represented by the following general formula (1).
[C1]

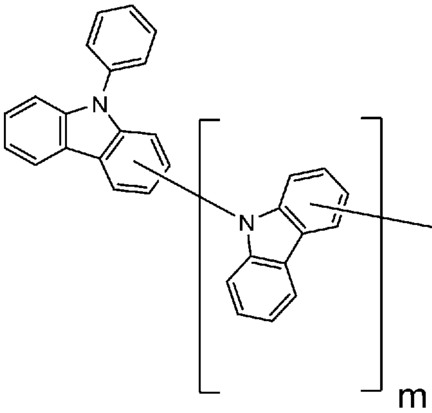

(1)

wherein m is the number of repetitions and represents an integer of 0 to 2. The deuteration ratio in the compound represented by the general formula (1) is 50% or more, and preferably 80% or more.
The compound represented by the general formula (1) preferably contains at least one bonding structure represented by the following formula (2) or formula (3). Some or all hydrogens in the compound represented by the following formula (2) or formula (3) are optionally replaced by deuteriums.
[C2]

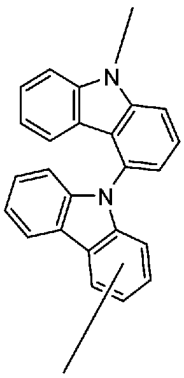

(2)

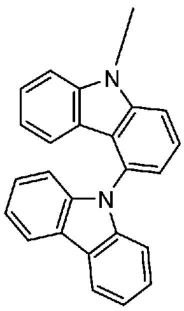

(3)

The present invention is a mixed composition for an organic electroluminescent device in which the material for an organic electroluminescent device represented by the general formula (1) and a cyclic azine compound are mixed.
The cyclic azine compound is preferably represented by any one of the following general formulas (4) to (10), more preferably represented by any one of the general formulas (4), (5), (6), and (10), and further preferably represented by any one of the general formulas (5), (6), and (10).
[C3]

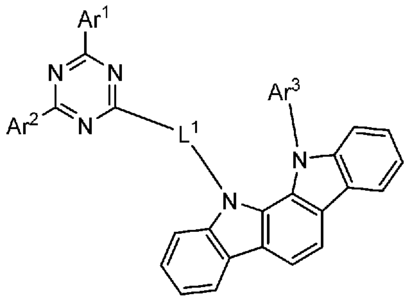
(4)

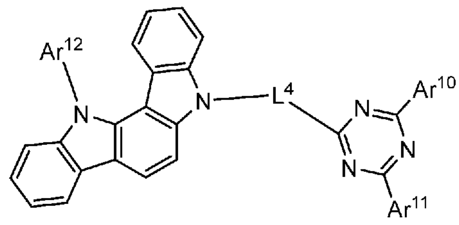
(5)

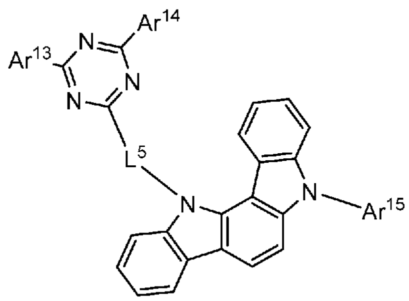
(6)

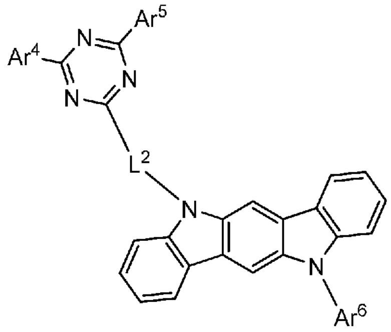
(7)

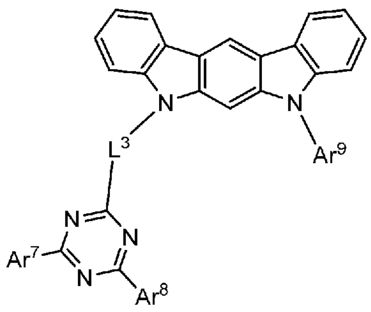
(8)

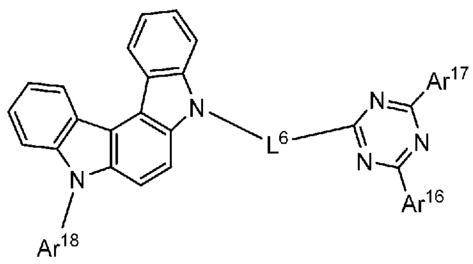
(9)

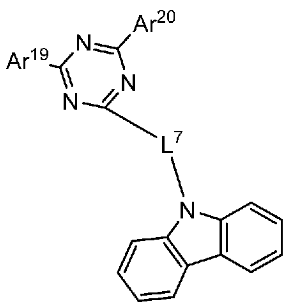
(10)

In the formula, $Ar^1$ to $Ar^{18}$ each independently represent a substituted or unsubstituted aromatic hydrocarbon group having 6 to 20 carbon atoms, a substituted or unsubstituted aromatic heterocyclic group having 2 to 20 carbon atoms, or a substituted or unsubstituted linked aromatic group formed by linking 2 to 3 aromatic groups selected from the aromatic hydrocarbon group and the aromatic heterocyclic group.

$Ar^{19}$ and $Ar^{20}$ each independently represent a substituted or unsubstituted aromatic hydrocarbon group having 6 to 20 carbon atoms, a substituted or unsubstituted aromatic heterocyclic group having 2 to 17 carbon atoms, or a substituted or unsubstituted linked aromatic group formed by linking 2 to 3 aromatic groups selected from the group consisting of the aromatic hydrocarbon group and the aromatic heterocyclic group.

$L^1$ to $L^7$ each independently represent a single bond, or a substituted or unsubstituted phenyl group, and preferably a single bond.

Hydrogen in the compounds represented by the general formulas (4) to (10) is optionally replaced by deuterium.

The present invention is an organic electroluminescent device including one or more organic layers between an anode and a cathode opposite to each other, wherein at least one of the organic layers contains the above material for an organic electroluminescent device or mixed composition for an organic electroluminescent device.

In the organic electroluminescent device of the present invention, it is preferred that at least one organic layer of the organic layers be a light emitting layer and the organic electroluminescent device contain a thermally activated delayed fluorescence material in the light emitting layer.
In the organic electroluminescent device of the present invention, it is preferred that at least one organic layer of the organic layers be a light emitting layer and the organic electroluminescent device contain a phosphorescence material in the light emitting layer.
In the organic electroluminescent device of the present invention, it is preferred that at least one organic layer of the organic layers be a light emitting layer and the light emitting layer contain the material for an organic electroluminescent device or mixed composition for an organic electroluminescent device of the present invention as the host material.
In addition, the present invention is a method for producing an organic electroluminescent device having a plurality of organic layers between an anode and a cathode, wherein at least one of the organic layers is a light emitting layer, the method preferably including producing the light emitting layer by providing a mixed composition for an organic electroluminescent device of the present invention and depositing the mixed composition from a deposition source.

Advantageous Effects of Invention

According to the present invention, a practically useful organic EL device having a low driving voltage, high emission efficiency, and long lifetime characteristics can be obtained.

Brief Description of Drawing

[Figure 1] Figure 1 is a schematic cross-sectional view of a structure example of the organic EL device used in the present invention.

Description of Embodiments

The compound represented by the general formula (1) in the present invention will be described in detail.
m is the number of repetitions and represents an integer of 0 to 2. m preferably represents an integer of 1 to 2, and more preferably an integer of 2.
Hydrogen in the compound represented by the general formula (1) is replaced by deuterium, and the deuteration ratio is 50% or more. The deuteration ratio is preferably 80% or more, and more preferably 90% or more.
The compound represented by the general formula (1) preferably contains at least one bonding structure represented by the general formula (2) or formula (3). The general formula (1) preferably has no substituent.
Conventional compounds having a carbazole backbone do not necessarily have sufficient performance as the light emitting device material. For example, it is known that compounds having a 4-(9-carbazolyl)carbazolecarbazole backbone as disclosed in Patent Literatures 3 to 6 exhibit high emission efficiency by being used as a hole-transporting host of a phosphorescence device. However, the lifetime characteristics of the organic EL device decrease in a blue light emitting device in which a phosphorescence material or a TADF luminescent material is used as a luminescent material. In this regard, the present inventors have focused on the fact that replacing hydrogen in the conventional carbazole compound by deuterium increases bond dissociation energy and improves the durability of the compound, and considered that the lifetime characteristics of the organic EL device are improved by deuterating hydrogen while maintaining high emission efficiency of the conventional carbazole compound. In addition, the present inventors have considered that, when the carbazole compound is deuterated, lifetime characteristics may vary depending on the number of introductions and the position to be introduced, and thus the invention of the compound represented by the general formula (1) has achieved.
The deuteration ratio in the general formula (1) represents the deuteration ratio of hydrogen on the carbazole ring and hydrogen on the benzene ring. That is, when the deuteration ratio is described specifically, a deuteration ratio of the following exemplified compound 1-1 of 50% means that 10 of 20 hydrogens are replaced by deuteriums on average. The compound represented by the general formula (1) may be a single compound or may be a mixture of compounds having a different deuteration ratio.
Specific examples of the material for an organic electroluminescent device represented by the general formula (1) are shown below, but the materials are not limited to these exemplified compounds. D in exemplified compounds represents deuterium, and n represents the number of deuterium.

[C4]

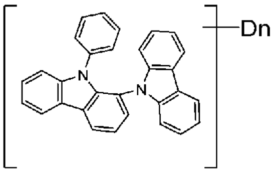

Dn (n = 10~20)

1-1

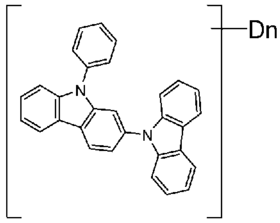

Dn (n =10~20)

1-2

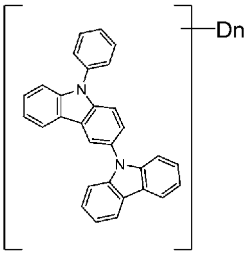

Dn (n = 10~20)

1-3

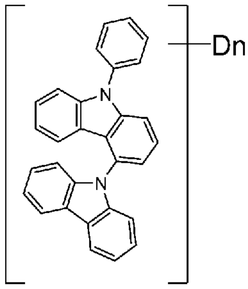

Dn (n = 10~20)

1-4

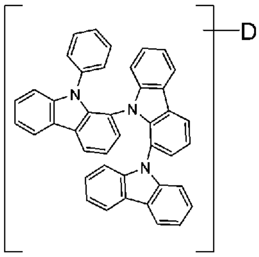

Dn (n = 14~27)

1-5

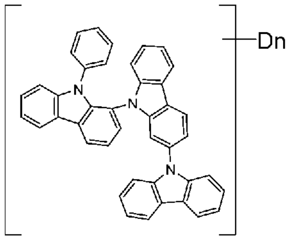

Dn (n =14~27)

1-6

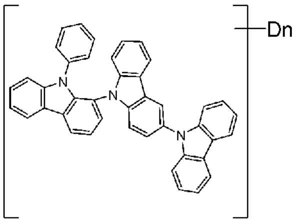

Dn (n =14~27)

1-7

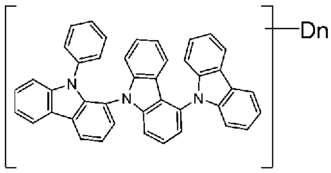

Dn (n = 14~27)

1-8

[C5]

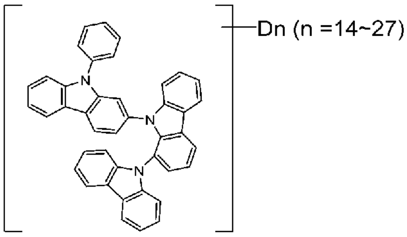

1-9

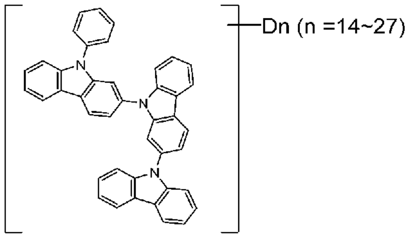

1-10

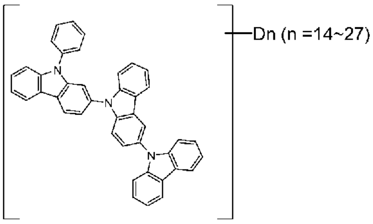

1-11

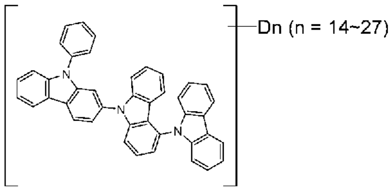

1-12

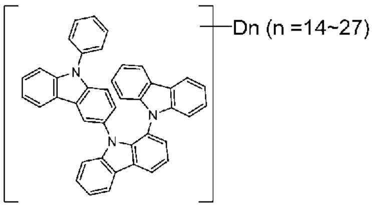

1-13

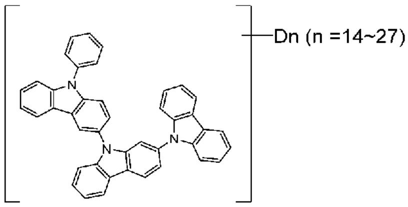

1-14

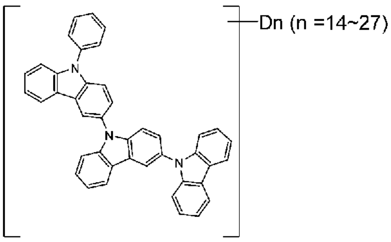

1-15

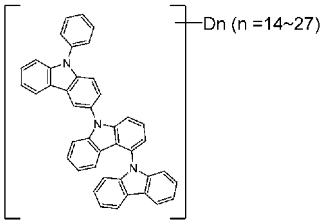

1-16

[C6]

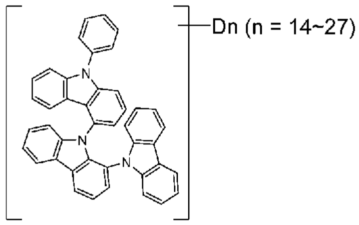

1-17

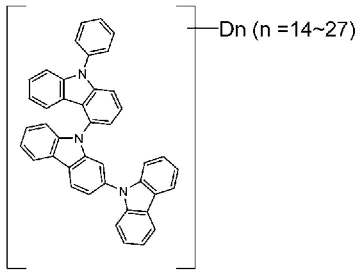

1-18

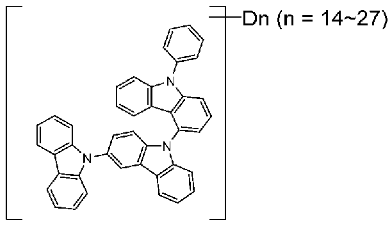

1-19

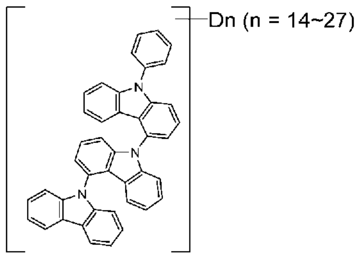

1-20

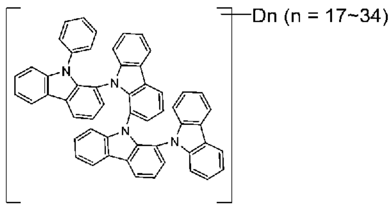

1-21

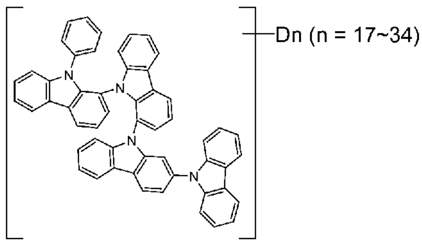

1-22

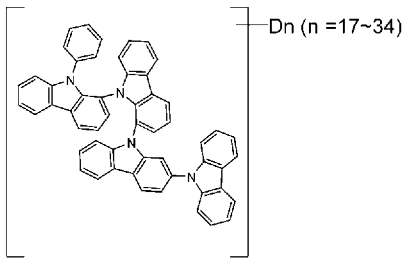

1-23

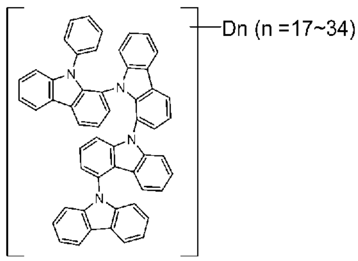

1-24

[C7]

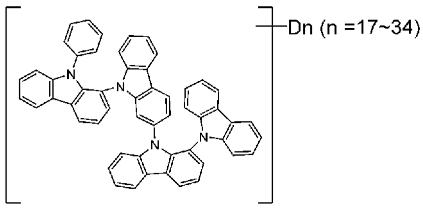

1-25

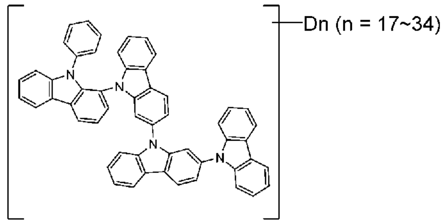

1-26

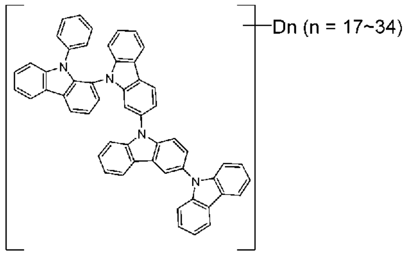

1-27

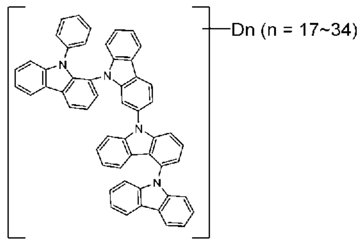

1-28

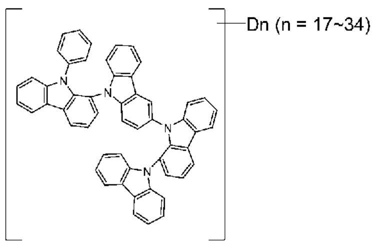

1-29

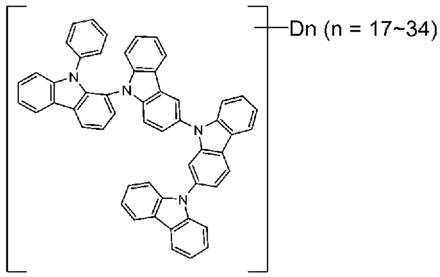

1-30

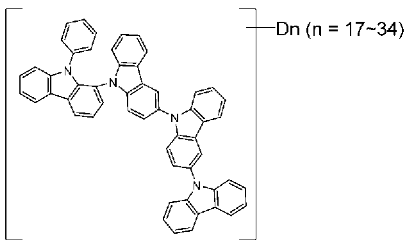

1-31

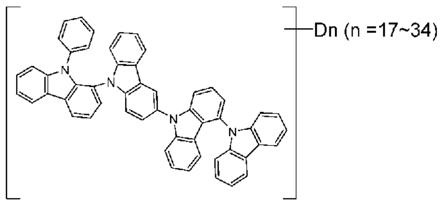

1-32

[C8]

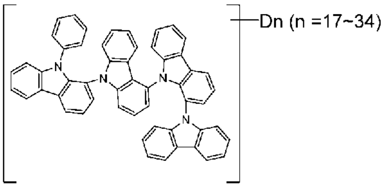
1-33

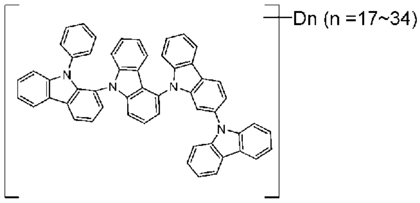
1-34

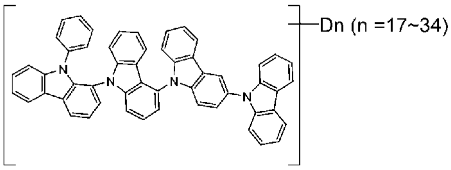
1-35

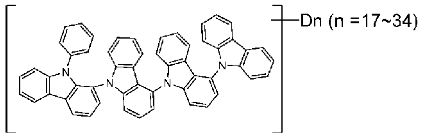
1-36

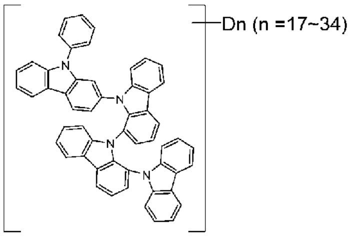
1-37

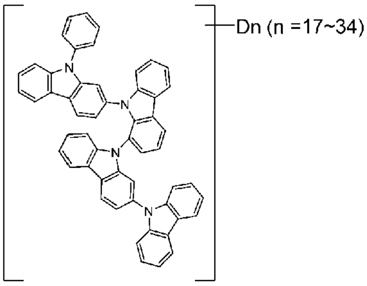
1-38

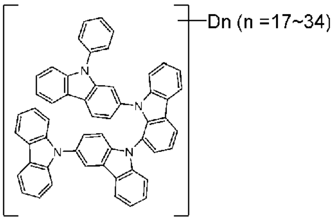
1-39

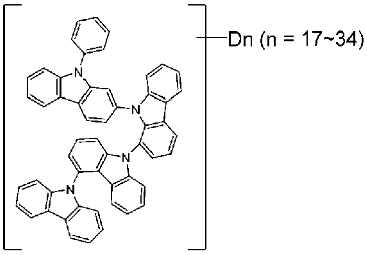
1-40

[C9]

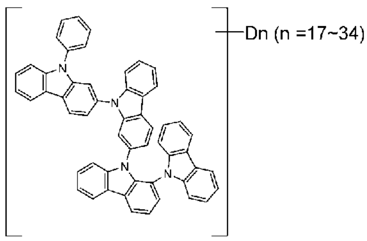
1-41

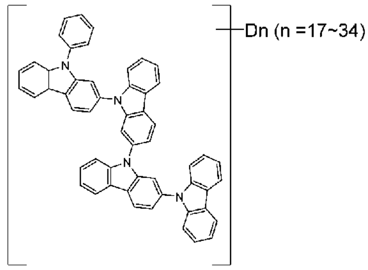
1-42

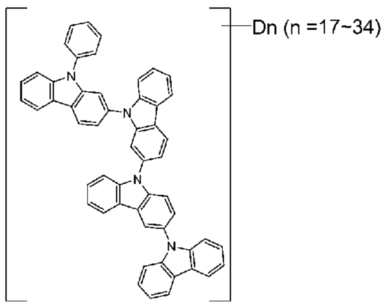
1-43

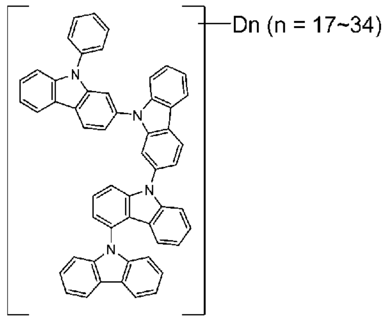
1-44

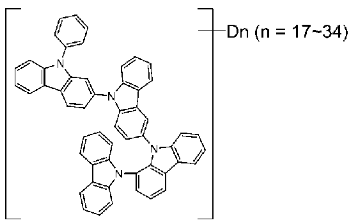
1-45

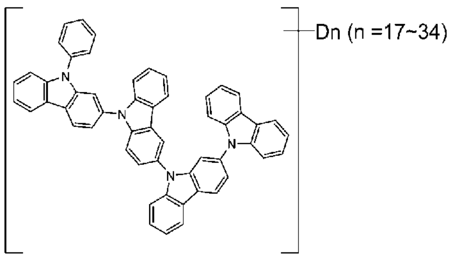
1-46

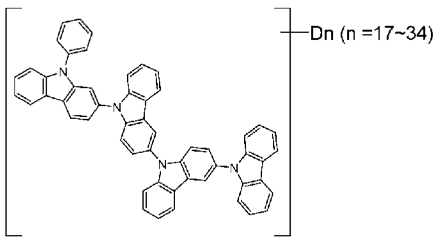
1-47

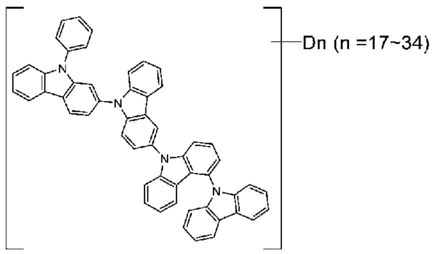
1-48

[C10]

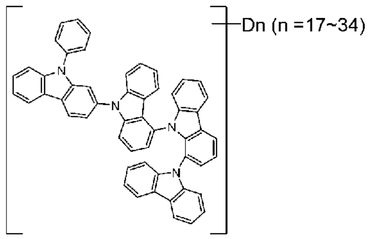

1-49

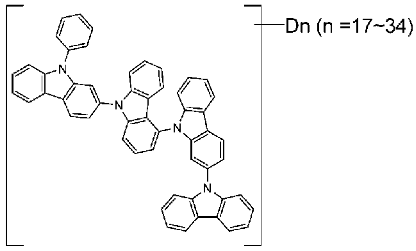

1-50

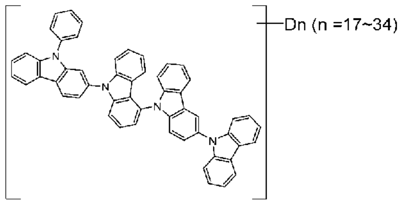

1-51

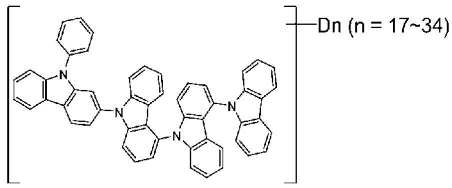

1-52

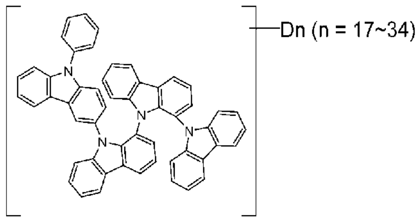

1-53

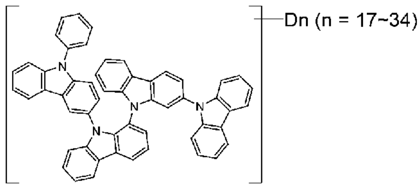

1-54

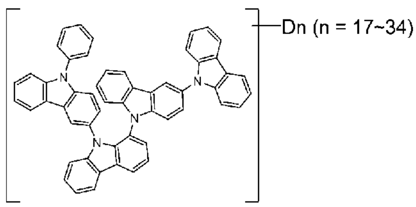

1-55

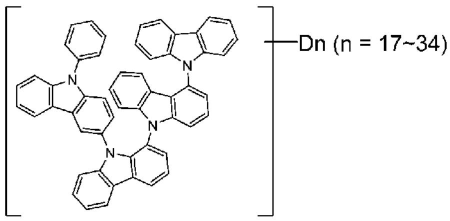

1-56

[C11]

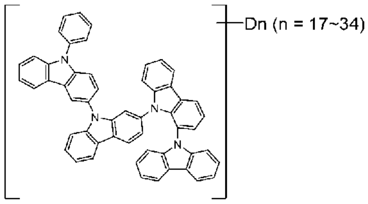


1-57

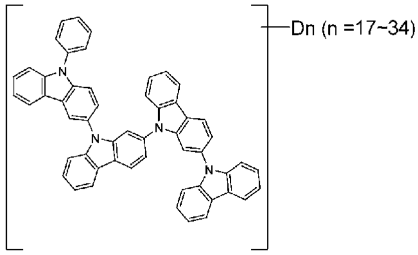


1-58

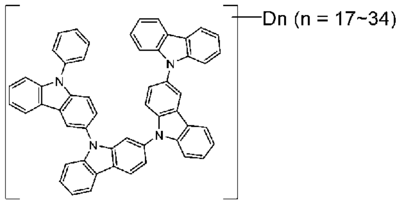


1-59

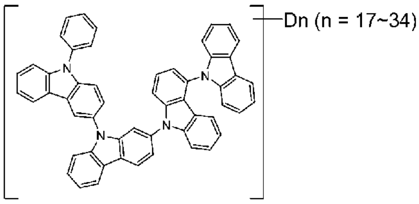


1-60

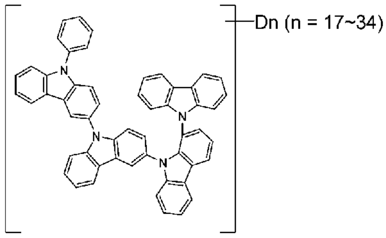


1-61

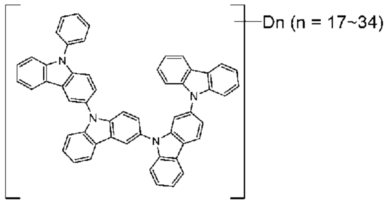


1-62

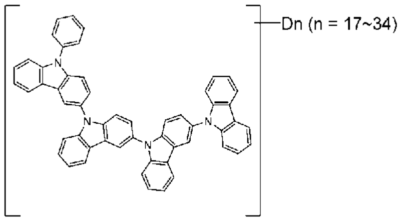


1-63

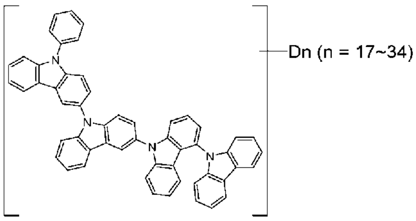


1-64

[C12]

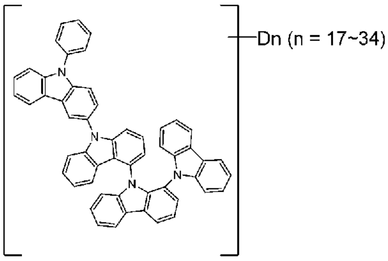

1-65

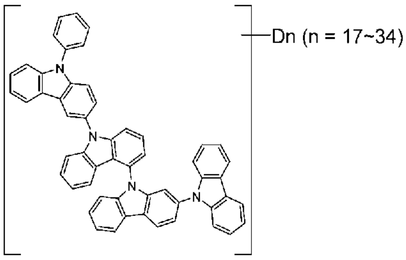

1-66

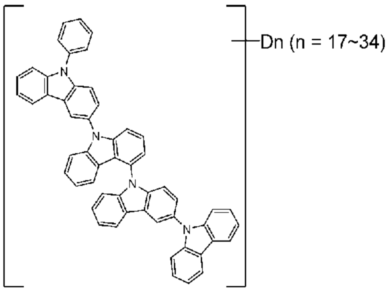

1-67

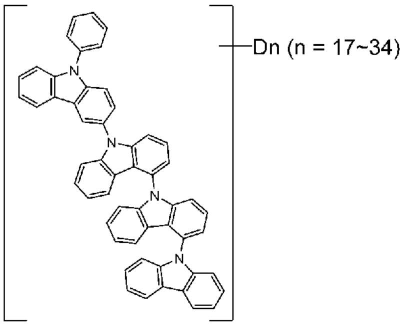

1-68

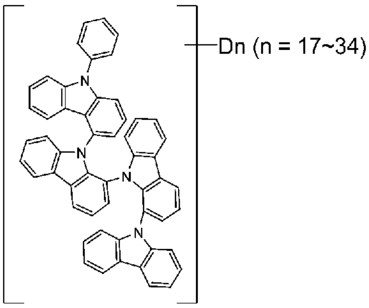

1-69

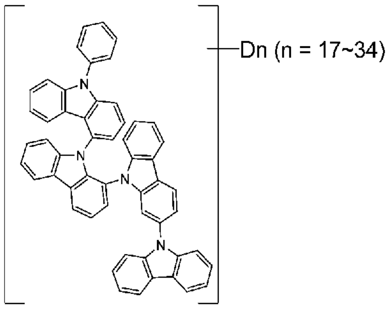

1-70

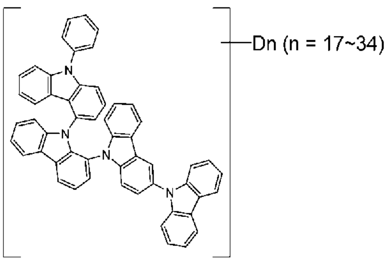

1-71

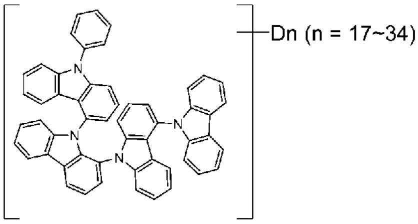

1-72

[C13]

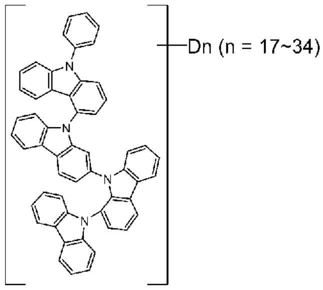
1-73

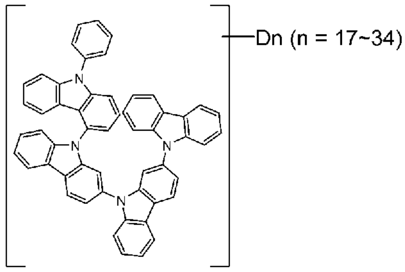
1-74

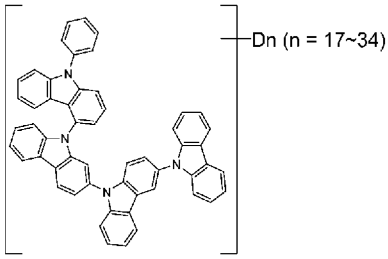
1-75

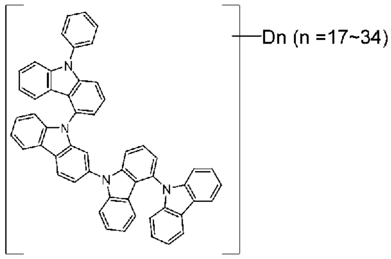
1-76

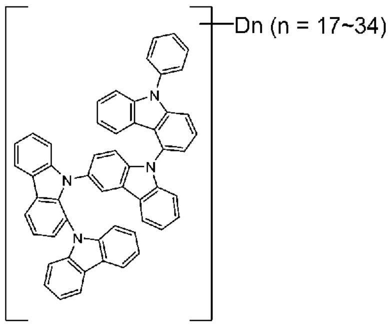
1-77

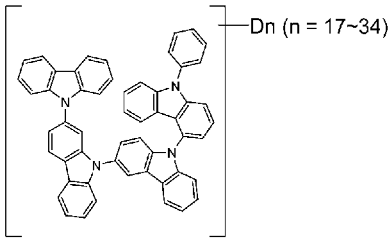
1-78

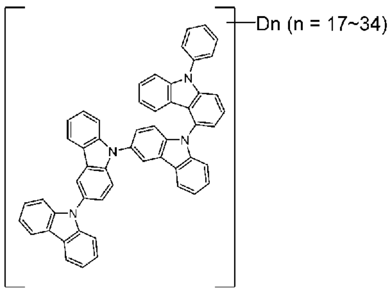
1-79

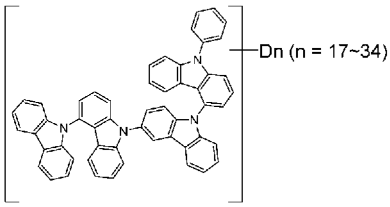
1-80

[C14]

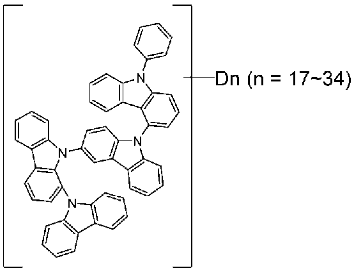
1-81

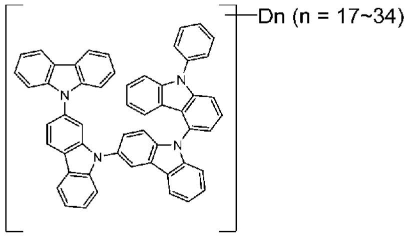
1-82

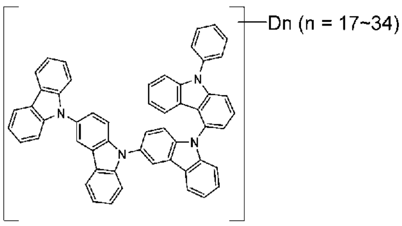
1-83

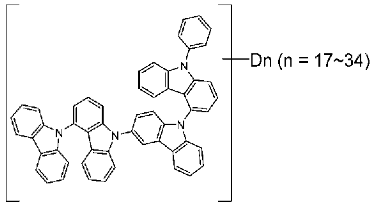
1-84

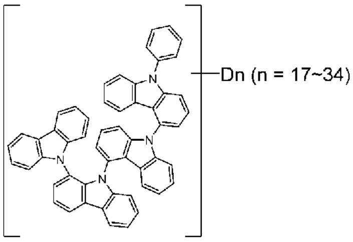
1-85

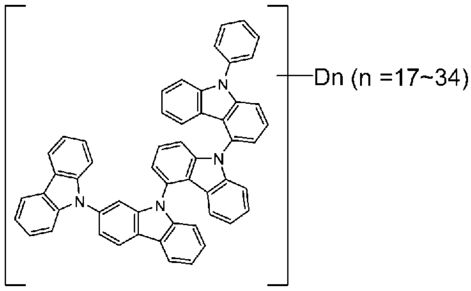
1-86

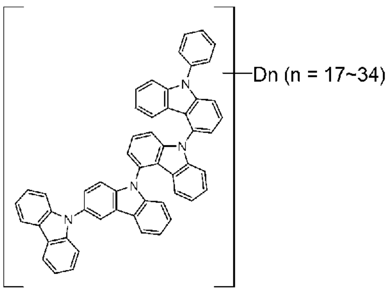
1-87

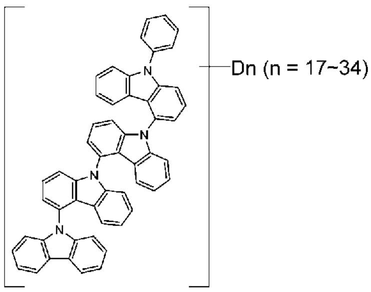
1-88

The method for deuterating hydrogen in the compound represented by the general formula (1) is not particularly limited, and deuterium can be prepared by using a deuterated compound as a reaction raw material or a precursor compound, or obtaining a non-deuterated compound of the compound represented by the general formula (1), and then treating the non-deuterated compound with a deuteration solvent (such as deuterated benzene) in the presence of a Lewis acid H/D exchange catalyst (such as aluminum(III) chloride, ethylaluminum chloride, or an acid such as trifluoromethanesulfonic acid, trifluoroacetic acid, or deuterated trifluoroacetic acid).

The deuteration ratio can be determined by mass spectrometry. In the mass spectrometry of the deuterated compound represented by the general formula (1), a molecular ion peak value having the highest intensity is measured as the molecular weight of the deuterated compound. The molecular weight of a non-deuterated compound corresponding to the deuterated compound is measured in the same manner, and the difference between the molecular weight of the deuterated compound and the molecular weight of the non-deuterated compound is determined, thereby allowing the number of replaced deuterium to be calculated. The deuteration ratio can be determined by calculating the proportion of the total number of deuterium to the total number of deuterium and hydrogen in the deuterated compound.

The mixed composition for an organic electroluminescent device in the present invention will be described in detail.

By mixing the compound represented by the general formula (1) and the cyclic azine compound, the mixed composition for an organic electroluminescent device can be obtained.

Specific examples of the cyclic azine include pyridine, pyrimidine, triazine, pyridazine, and pyrazine. The cyclic azine is preferably pyridine or triazine, and more preferably triazine.

The mixed composition may be produced by mixing the compound represented by the general formula (1) and the cyclic azine compound in powder state, by heating and melting them under reduced pressure or in an inert gas atmosphere such as nitrogen and then melt-mixing them, or by sublimating the compounds to be mixed together. The mixed composition may be produced as a thin film by vapor deposition or the like. The mixed composition may be contained in separate layers in the device, and for example, the compound represented by the general formula (1) may be contained in an electron blocking layer and a cyclic azine compound may be contained in a light emitting layer.

As for the mixing ratio (weight ratio) of the compound represented by the general formula (1) and the cyclic azine compound in the mixed composition, the proportion of the compound represented by the general formula (1) to the total amount of the compound represented by the general formula (1) and the cyclic azine compound is 40 to 90 wt%, preferably 50 to 90 wt%, and more preferably 60 to 90 wt%.

The cyclic azine compound is preferably represented by any one of the general formulas (4) to (10), and more preferably represented by any one of the general formulas (4) to (6), and (10).

$Ar^1$ to $Ar^{18}$ each independently represent a substituted or unsubstituted aromatic hydrocarbon group having 6 to 20 carbon atoms, a substituted or unsubstituted aromatic heterocyclic group having 2 to 20 carbon atoms, or a substituted or unsubstituted linked aromatic group formed by linking 2 to 3 aromatic groups selected from the group consisting of the aromatic hydrocarbon group and the aromatic heterocyclic group. $Ar^1$ to $Ar^{18}$ preferably each independently represent a substituted or unsubstituted aromatic hydrocarbon group having 6 to 15 carbon atoms, a substituted or unsubstituted aromatic heterocyclic group having 2 to 15 carbon atoms, or a substituted or unsubstituted linked aromatic group formed by linking 2 to 3 aromatic groups selected from the group consisting of the aromatic hydrocarbon group and the aromatic heterocyclic group. $Ar^1$ to $Ar^{18}$ more preferably each independently represent a substituted or unsubstituted aromatic hydrocarbon group having 6 to 15 carbon atoms, or a substituted or unsubstituted linked aromatic group formed by linking 2 to 3 aromatic groups selected from the group consisting of the aromatic hydrocarbon group.

Specific examples of the unsubstituted $Ar^1$ to $Ar^{18}$ include a group produced from benzene, naphthalene, acenaphthene, acenaphthylene, azulene, anthracene, chrysene, pyrene, phenanthrene, triphenylene, fluorene, benzo[a] anthracene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzoisothiazole, benzothiadiazole, purine, pyranone, coumarin, isocoumarin, chromone, dibenzofuran, dibenzothiophene, dibenzoselenophene, carbazole, or indolocarbazole, or a linked aromatic group formed by linking 2 to 3 of these aromatic groups. Preferred examples thereof include a group produced from benzene, naphthalene, acenaphthene, acenaphthylene, azulene, anthracene, phenanthrene, fluorene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzoisothiazole, benzothiadiazole, purine, pyranone, coumarin, isocoumarin, chromone, dibenzofuran, dibenzothiophene, dibenzoselenophene, or carbazole, or a linked aromatic group formed by linking 2 to 3 of these aromatic groups. More preferred examples thereof include a group produced from benzene, naphthalene, acenaphthene, acenaphthylene, azulene, anthracene, phenanthrene, or fluorene, or a linked aromatic group formed by linking 2 to 3 of these aromatic groups.

As used herein, each of the unsubstituted aromatic hydrocarbon group, the aromatic heterocyclic group, or the linked aromatic group may have a substituent, except for the compound represented by the general formula (1). In the case where the above groups have a substituent, the substituent is preferably a deuterium, a halogen, a cyano group, an alkyl group having 1 to 10 carbon atoms, a triarylsilyl group having 9 to 30 carbon atoms, an alkenyl group having 2 to 5 carbon atoms, an alkoxy group having 1 to 5 carbon atoms, or a diarylamino group having 12 to 44 carbon atoms.

The number of substituents is 0 to 5, and preferably 0 to 2. When the aromatic hydrocarbon group, the aromatic heterocyclic group, or the linked aromatic group has a substituent, the number of carbon atoms of the substituent is not included in the calculation of the number of carbon atoms. However, it is preferred that the total number of carbon atoms including the number of carbon atoms of the substituent satisfy the above range.

Specific examples of the above substituent include deuterium, cyano, bromo, fluorine, methyl, ethyl, propyl, i-propyl, butyl, t-butyl, pentyl, cyclopentyl, hexyl, cyclohexyl, heptyl, octyl, nonyl, decyl, triphenylsilyl, biphenyldiphenylsilyl, bisbiphenylphenylsilyl, trisbiphenylsilyl, vinyl, propenyl, butenyl, pentenyl, methoxy, ethoxy, propoxy, butoxy, pentoxy, diphenylamino, naphthylphenylamino, dinaphthylamino, dianthranilamino, diphenanthrenylamino, and dipyrenylamino. Preferred examples thereof include deuterium, cyano, methyl, ethyl, propyl, butyl, pentyl, hexyl, heptyl, octyl, vinyl, propenyl, butenyl, pentenyl, methoxy, ethoxy, propoxy, butoxy, and pentoxy.

As used herein, the linked aromatic group refers to an aromatic group linked by bonding the aromatic rings of two

or more aromatic groups by a single bond. These linked aromatic groups may be linear or branched. The linking position when benzene rings are linked to each other may be any of ortho, meta, and para, but the para linkage or the meta linkage is preferred. The aromatic group may be an aromatic hydrocarbon group or an aromatic heterocyclic group, and a plurality of aromatic groups may be the same or different from each other.

$Ar^{19}$ and $Ar^{20}$ each independently represent a substituted or unsubstituted aromatic hydrocarbon group having 6 to 20 carbon atoms, a substituted or unsubstituted aromatic heterocyclic group having 2 to 17 carbon atoms, or a substituted or unsubstituted linked aromatic group formed by linking 2 to 3 aromatic groups selected from the group consisting of the aromatic hydrocarbon group and the aromatic heterocyclic group. $Ar^{19}$ and $Ar^{20}$ preferably each independently represent a substituted or unsubstituted aromatic hydrocarbon group having 6 to 15 carbon atoms, a substituted or unsubstituted aromatic heterocyclic group having 2 to 15 carbon atoms, or a substituted or unsubstituted linked aromatic group formed by linking 2 to 3 aromatic groups selected from the group consisting of the aromatic hydrocarbon group and the aromatic heterocyclic group. $Ar^{19}$ and $Ar^{20}$ more preferably each independently represent a substituted or unsubstituted aromatic hydrocarbon group having 6 to 15 carbon atoms, or a substituted or unsubstituted linked aromatic group formed by linking 2 to 3 aromatic groups selected from the group consisting of the aromatic hydrocarbon group.

Specific examples of the unsubstituted $Ar^{19}$ and $Ar^{20}$ include a group produced from benzene, naphthalene, acenaphthene, acenaphthylene, azulene, anthracene, chrysene, pyrene, phenanthrene, triphenylene, fluorene, benzo[a]anthracene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzoisothiazole, benzothiadiazole, purine, pyranone, coumarin, isocoumarin, chromone, dibenzofuran, dibenzothiophene, dibenzoselenophene, or carbazole, or a linked aromatic group formed by linking 2 to 3 of these aromatic groups. Preferred examples thereof include a group produced from benzene, naphthalene, acenaphthene, acenaphthylene, azulene, anthracene, phenanthrene, fluorene, pyridine, pyrimidine, triazine, thiophene, isothiazole, thiazole, pyridazine, pyrrole, pyrazole, imidazole, triazole, thiadiazole, pyrazine, furan, isoxazole, quinoline, isoquinoline, quinoxaline, quinazoline, thiadiazole, phthalazine, tetrazole, indole, benzofuran, benzothiophene, benzoxazole, benzothiazole, indazole, benzimidazole, benzotriazole, benzoisothiazole, benzothiadiazole, purine, pyranone, coumarin, isocoumarin, chromone, dibenzofuran, dibenzothiophene, dibenzoselenophene, or carbazole, or a linked aromatic group formed by linking 2 to 3 of these aromatic groups. More preferred examples thereof include a group produced from benzene, naphthalene, acenaphthene, acenaphthylene, azulene, anthracene, phenanthrene, or fluorene, or a linked aromatic group formed by linking 2 to 3 of these aromatic groups.

$L^1$ to $L^7$ each independently represent a single bond or a substituted or unsubstituted phenyl group. $L^1$ to $L^7$ each independently preferably represent a single bond.

Hydrogen in the compounds represented by the general formulas (4) to (10) is optionally replaced by deuterium.

That is, some or all hydrogen on the carbazole rings and hydrogen on the indolocarbazole rings in the compounds represented by the general formulas (4) to (10), hydrogen on the aromatic rings of $Ar^1$ to $Ar^{20}$ and $L^1$ to $L^7$, and hydrogen in the substituents may be deuterium. The compounds represented by the general formulas (4) to (10) may be single compounds or mixtures of compounds having a different deuteration ratio.

Specific examples of the materials for an organic electroluminescent device represented by the general formulas (4) to (10) are shown below, but the materials are not limited to these exemplified compounds.

[C15]

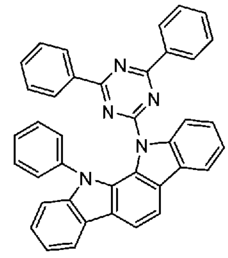
2－1

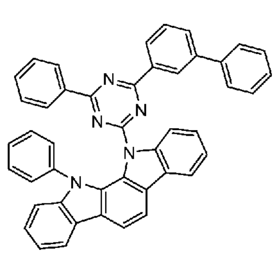
2－2

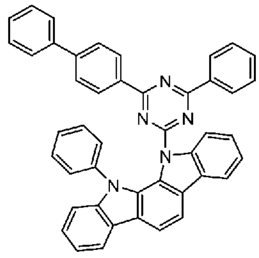
2－3

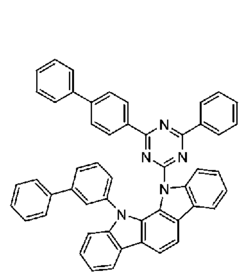
2－4

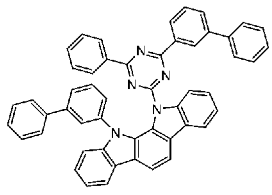
2－5

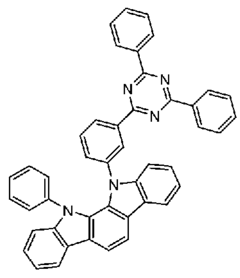
2－6

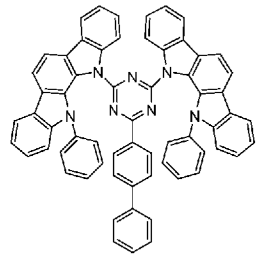
2－7

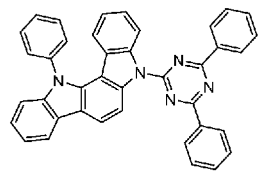
2－8

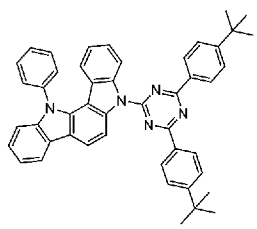
2－9

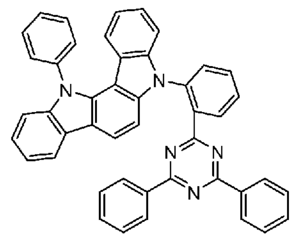
2－1 0

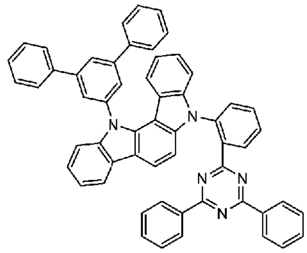
2－1 1

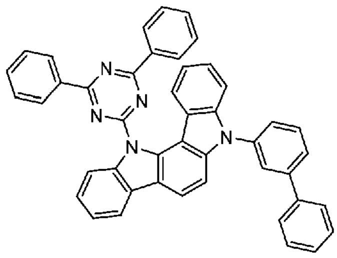
2－1 2

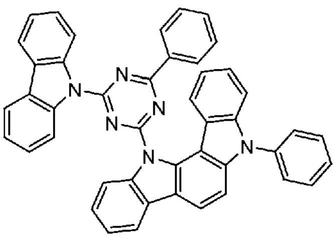
2－1 3

[C16]

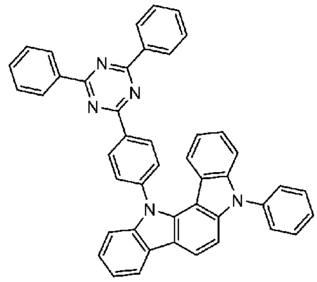
2－14
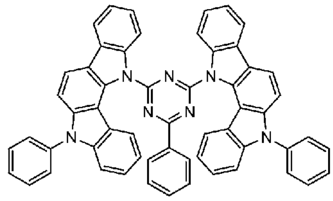
2－15

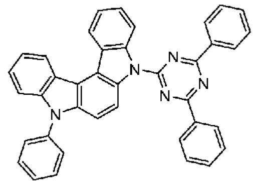
2－16
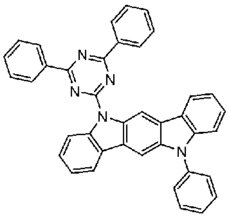
2－17
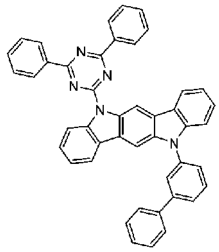
2－18

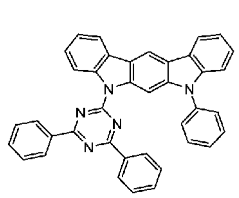
2－19
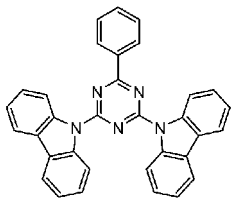
2－20
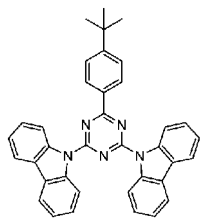
2－21

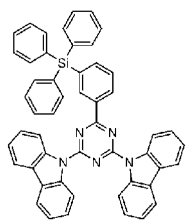
2－22
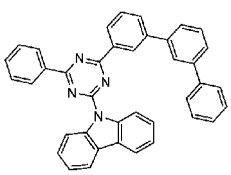
2－23
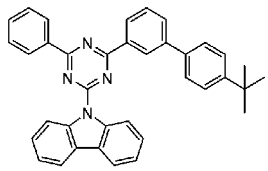
2－24

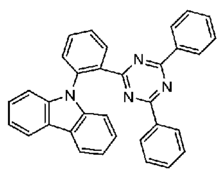
2－25
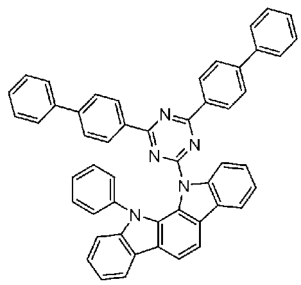
2－26

[C17]

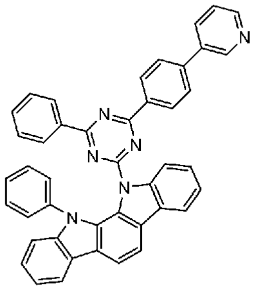

2－27

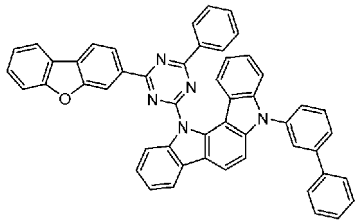

2－28

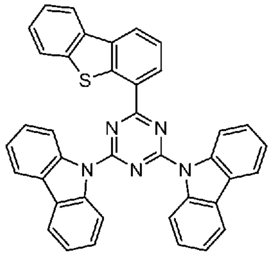

2－29

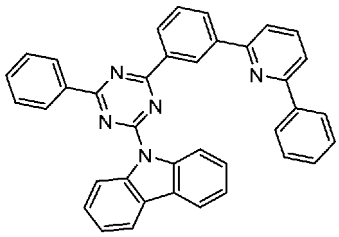

2－30

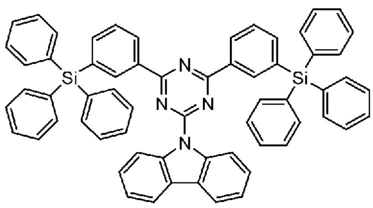

2－31

A practically excellent organic EL device having high emission efficiency and long lifetime characteristics can be provided by containing the material for an organic electroluminescent device represented by the general formula (1), or the mixed composition for an organic electroluminescent device obtained by mixing the compound represented by the general formula (1) and the cyclic azine compound in an organic layer.

The organic EL device is preferably an organic EL device in which at least one organic layer is a light emitting layer, and which contains a thermally activated delayed fluorescence material or a phosphorescence material in the light emitting layer, and more preferably an organic EL device containing a thermally activated delayed fluorescence material in the light emitting layer.

An excellent organic EL device can be provided by containing at least one host material together with a thermally activated delayed fluorescence material or a phosphorescence material in the light emitting layer as needed, and the at least one host material is preferably the material for an organic electroluminescent device or the mixed composition for an organic electroluminescent device.

The light emitting layer of the organic EL device can be produced by depositing the mixed composition for an organic electroluminescent device that is prepared by mixing powder or heating and melting powder in advance from a deposition source.

Next, the structure of the organic EL device of the present invention will be described with reference to the drawing, but the structure of the organic EL device of the present invention is not limited thereto.

Figure 1 shows a cross-sectional view of a structure example of a typical organic EL device used in the present invention. Reference numeral 1 denotes a substrate, reference numeral 2 denotes an anode, reference numeral 3 denotes a hole injection layer, reference numeral 4 denotes a hole transport layer, reference numeral 5 denotes a light emitting layer, reference numeral 6 denotes an electron transport layer, and reference numeral 7 denotes a cathode. The organic EL device of the present invention has the anode, the light emitting layer, and the cathode as essential layers, but preferably has a hole injection layer, a hole transport layer, an electron transport layer, and an electron injection layer in addition to the essential layers, and may further have an electron blocking layer between the hole transport layer and the light emitting layer, and a hole blocking layer between the light emitting layer and the electron transport layer.

It is also possible to have a structure that is the reverse of the structure shown in Figure 1, that is, the cathode 7, the electron transport layer 6, the light emitting layer 5, the hole transport layer 4, the hole injection layer 3, and the anode 2 can be laminated on the substrate 1, in the order presented. Also, in this case, layers can be added or omitted, as necessary. In the organic EL device as described above, layers other than electrodes such as an anode and a cathode, the layers constituting a multilayer structure on a substrate, may be collectively referred to as an organic layer in some cases.

-Substrate-

The organic EL device of the present invention is preferably supported on a substrate. The substrate is not particularly limited and may be a substrate conventionally used for organic EL devices, and for example, a substrate made of glass, transparent plastic, or quartz can be used.

-Anode-

As the anode material in the organic EL device, a material made of a metal, alloy, or conductive compound having a high work function (4 eV or more), or a mixture thereof is preferably used. Specific examples of such an electrode material include metals such as Au, and conductive transparent materials such as CuI, indium tin oxide (ITO), $SnO_2$, and ZnO. An amorphous material capable of producing a transparent conductive film such as IDIXO ($In_2O_3$-ZnO) may also be used. As the anode, these electrode materials may be formed into a thin film by a method such as vapor deposition or sputtering, and then a pattern of a desired form may be formed by photolithography. Alternatively, when a highly precise pattern is not required (about 100 $\mu$m or more), a pattern may be formed through a mask of a desired form at the time of vapor deposition or sputtering of the above electrode materials. Alternatively, when a coatable material such as an organic conductive compound is used, a wet film forming method such as a printing method and a coating method can also be used. When light is extracted from the anode, the transmittance is desirably more than 10%, and the sheet resistance as the anode is preferably several hundred $\Omega$/square or less. The film thickness is selected within a range of usually 10 to 1,000 nm, and preferably 10 to 200 nm, although it depends on the material.

-Cathode-

On the other hand, a material made of a metal (referred to as an electron injection metal), alloy, or conductive compound having a low work function (4 eV or less) or a mixture thereof is used as the cathode material. Specific examples of such an electrode material include sodium, a sodium-potassium alloy, magnesium, lithium, a magnesium/copper mixture, a magnesium/silver mixture, a magnesium/aluminum mixture, a magnesium/indium mixture, an aluminum/aluminum oxide ($Al_2O_3$) mixture, indium, a lithium/aluminum mixture, and a rare earth metal. Among them, in terms of electron injection properties and durability against oxidation and the like, a mixture of an electron injection metal with a second metal that has a higher work function value than the electron injection metal and is stable, for example, a magnesium/silver mixture, a magnesium/aluminum mixture, a magnesium/indium mixture, an aluminum/aluminum oxide ($Al_2O_3$) mixture, a lithium/aluminum mixture, or aluminum is suitable. The cathode can be produced by forming a thin film from these cathode materials by a method such as vapor deposition and sputtering. The sheet resistance as the cathode is preferably several hundred $\Omega$/square or less, and the film thickness is selected within a range of usually 10 nm to 5 $\mu$m, and preferably 50 to 200 nm. To transmit the light emitted, either one of the anode and the cathode of the organic EL device is favorably transparent or translucent because light emission brightness is improved.

The above metal is formed on the cathode to have a film thickness of 1 to 20 nm, and then a conductive transparent material mentioned in the description of the anode is formed on the metal, so that a transparent or translucent cathode can be produced. By applying this process, a device in which both anode and cathode have transmittance can be produced.

-Light Emitting Layer-

The light emitting layer is a layer that emits light after holes and electrons respectively injected from the anode and the cathode are recombined to form exciton. The light emitting layer may be either a single layer or a plurality of layers, and each layer contains an organic light emitting dopant material and a host material.

Only one organic light emitting dopant or two or more organic light emitting dopants may be contained in the light emitting layer. The content of the organic light emitting dopant is preferably 0.1 to 50 wt%, and more preferably 0.1 to 40 wt% based on the host material.

When a phosphorescence dopant is used as the organic light emitting dopant material, a phosphorescence dopant containing an organic metal complex including at least one metal selected from the group consisting of ruthenium, rhodium, palladium, silver, rhenium, osmium, iridium, platinum, and gold is preferred. Specifically, iridium complexes described in J. Am. Chem. Soc. 2001, 123, 4304 and JP2013-530515 A and platinum complexes described in Adv. Mater. 2014, 26, 7116 and JP2018-2722 A are suitably used, but are not limited thereto.

The phosphorescence dopant material is not particularly limited, but specific examples thereof include the following.

[C18]

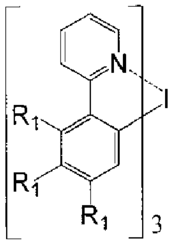

$R_1$:H, $CH_3$, $CF_3$, F

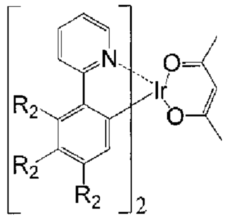

$R_2$:H, F

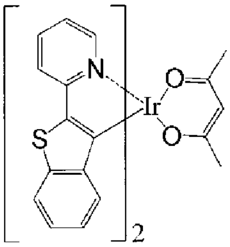
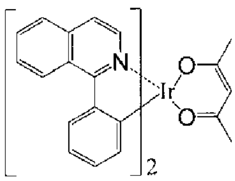
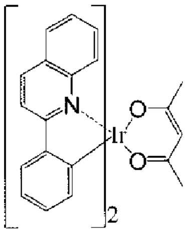
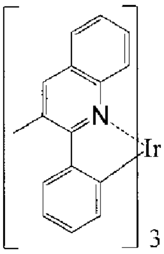
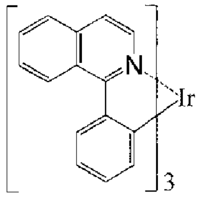

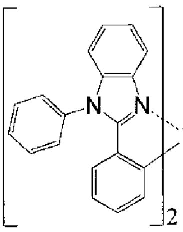
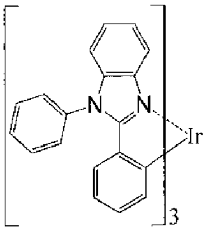
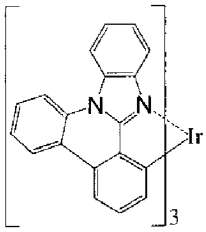
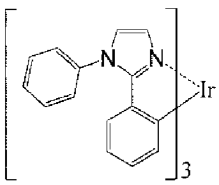

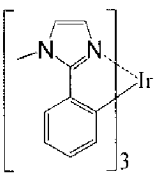
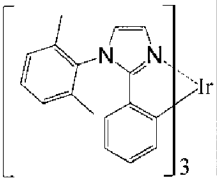
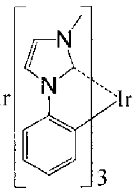
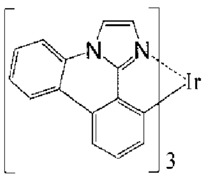

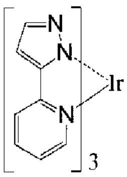
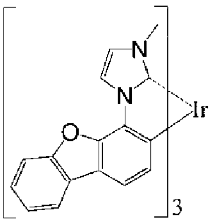
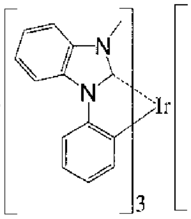
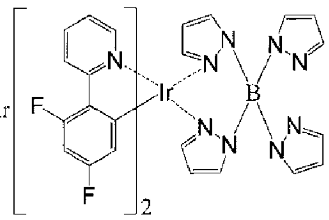

[C19]

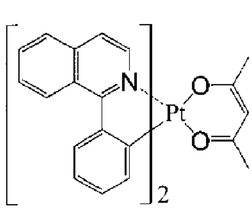

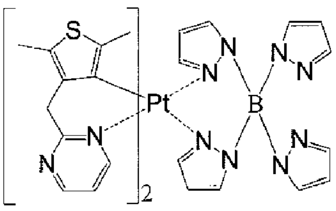

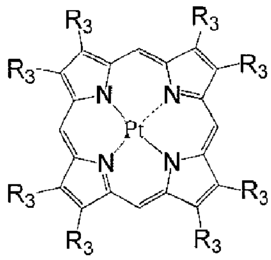

$R_3$: $CH_3$, $CH_2CH_3$

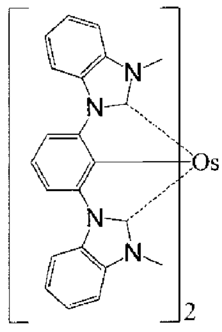

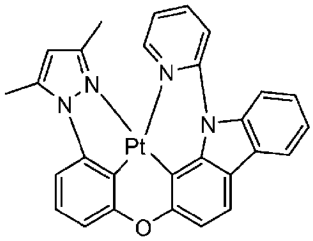

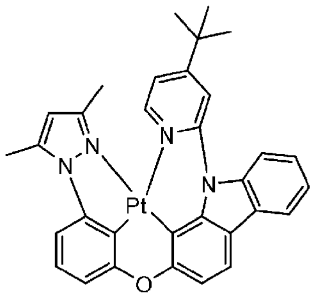

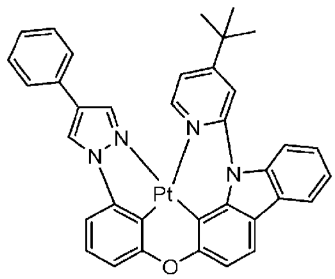

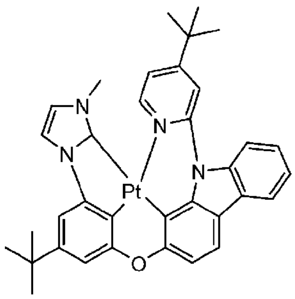

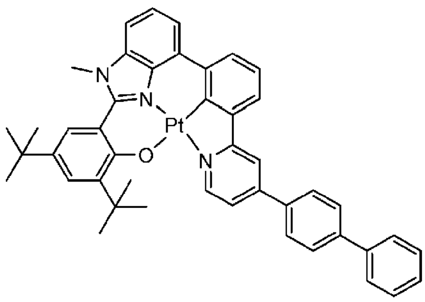

When a fluorescence dopant is used as the light emitting dopant material, examples of the fluorescence dopant include, but are not particularly limited to, fused polycyclic aromatic derivatives, styrylamine derivatives, fused ring amine derivatives, boron-containing compounds, pyrrole derivatives, indole derivatives, carbazole derivatives, and indolocarbazole derivatives. Among them, fused ring amine derivatives, boron-containing compounds, carbazole derivatives, and indolocarbazole derivatives are preferred. Examples of the fused ring amine derivatives include diaminepyrene derivatives, diaminochrysene derivatives, diaminoanthracene derivatives, diaminofluorenone derivatives, and diaminofluorene derivatives fused with one or more benzofuro backbones.

Examples of the boron-containing compounds include pyrromethene derivatives, and polycyclic aromatic compounds described in, for example, WO2015/102118.

The fluorescence dopant material is not particularly limited, but specific examples thereof include the following.

[C20]

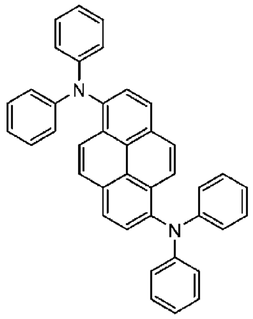

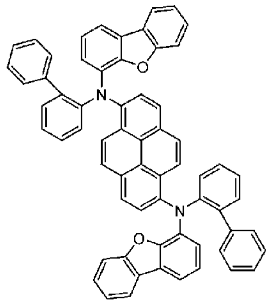

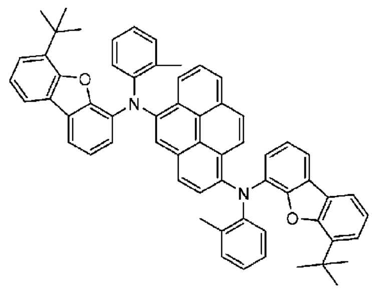

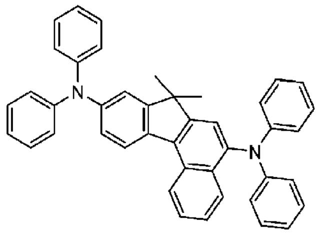

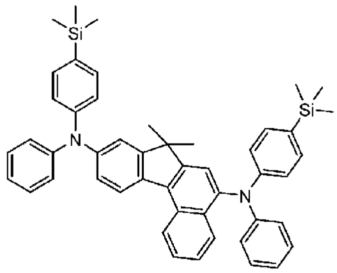

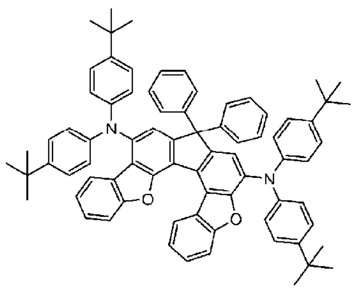

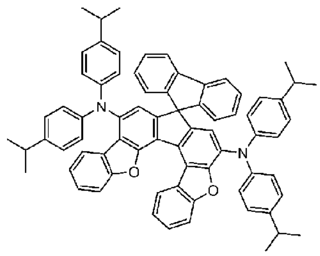

[C21]

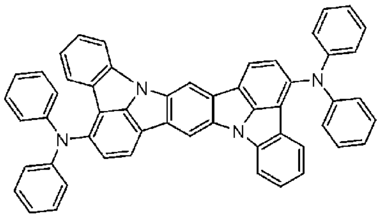
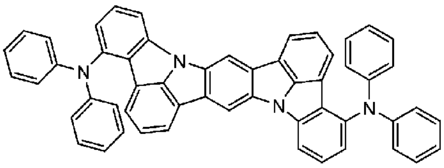
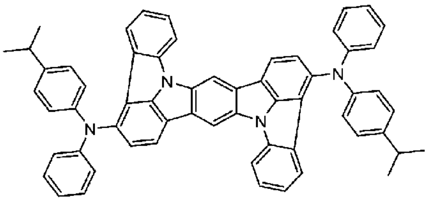
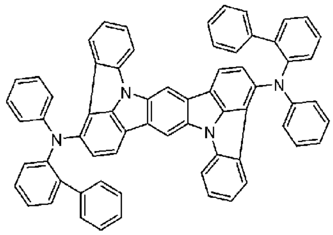
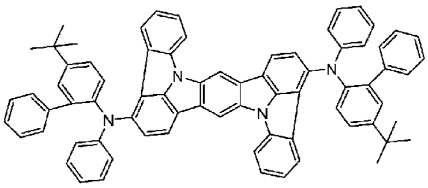
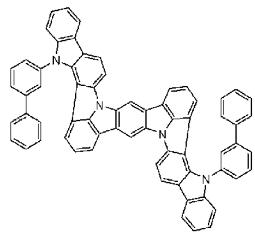
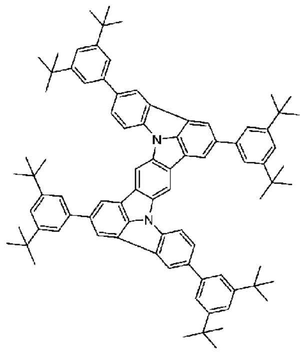
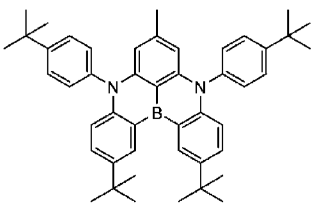
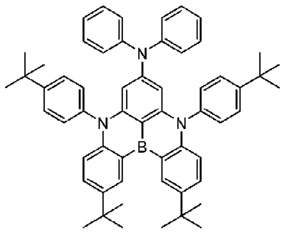
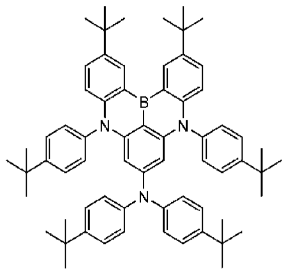

When a thermally activated delayed fluorescence dopant is used as the light emitting dopant material, examples of the thermally activated delayed fluorescence dopant include, but are not particularly limited to, metal complexes such as tin complexes and copper complexes, the indolocarbazole derivative described in WO2011/070963, the cyanobenzene derivative and carbazole derivative described in Nature 2012, 492, 234, the fenadine derivative, oxadiazole derivative, triazole derivative, sulfone derivative, phenoxazine derivative, and acridine derivative described in Nature Photonics 2014, 8, 326, and the polycyclic aromatic compound described in WO2015/102118.

The thermally activated delayed fluorescence dopant material is not particularly limited, but specific examples thereof include the following. A cyclic azine compound may be used as the thermally activated delayed fluorescence dopant material, but it is preferably not any of the compounds represented by the general formulas (4) to (10).

[C22]

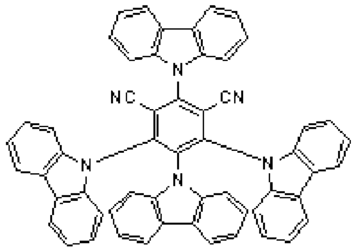
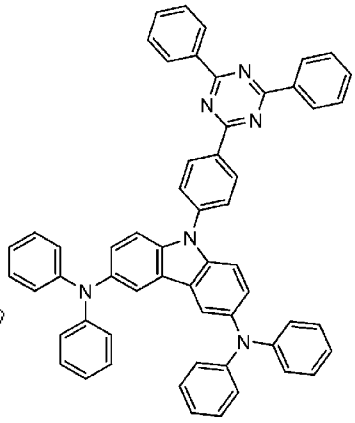
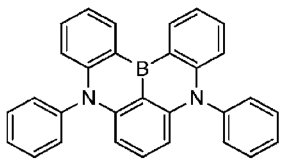
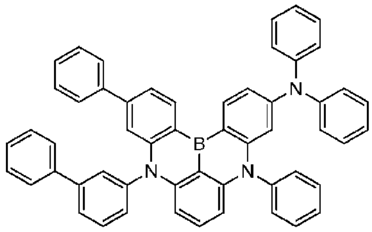
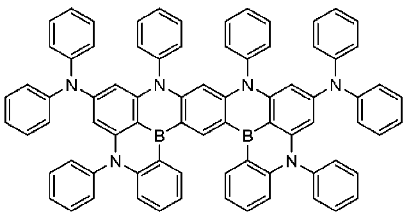
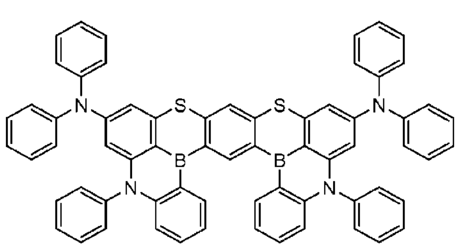
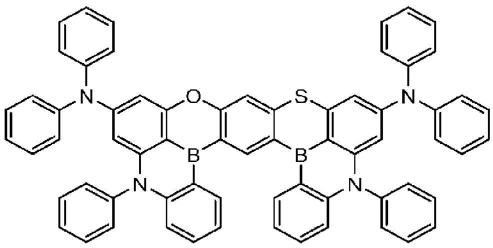
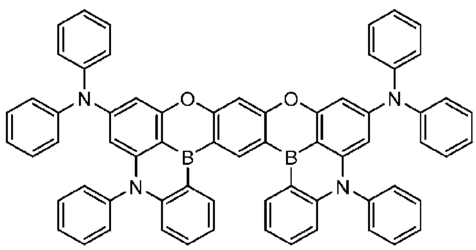

The compound represented by the general formula (1) is preferably used as the host material in the light emitting layer. When the compound represented by the general formula (1) is used in any of the organic layers other than the light emitting layer, the compound represented by the general formula (1) may be contained or may not be contained in the light emitting layer. At this time, a known host material used for a phosphorescence device or a fluorescence device can be used in the light emitting layer, and a cyclic azine compound is preferably used as the host material. A plurality of known host materials may be used in combination, or each of them may be used singly. A usable known host material is a compound having the ability to transport hole, the ability to transport electron, and a high glass transition temperature, and preferably has a higher triplet excited energy (T1) than the triplet excited energy (T1) of the light emitting dopant material. A TADF-active compound may also be used as the host material, and the TADF-active compound preferably has a difference ($\Delta EST = S1 - T1$) between the singlet excited energy (S1) and the triplet excited energy (T1), of 0.20 eV or less. When the compound represented by the general formula (1) is contained in the light emitting layer as the host material, other known host materials may be used in combination, and the cyclic azine compound may be preferably used in combination as the host material. Further, a plurality of known host materials may be used in combination, in addition to the cyclic azine compound.

Here, S1 and T1 are measured as follows.

A sample compound (thermally activated delayed fluorescence material) is deposited on a quartz substrate by a

vacuum deposition method under conditions of a degree of vacuum of $10^{-4}$ Pa or less to form a deposition film having a thickness of 100 nm. For S1, the emission spectrum of this deposition film is measured, a tangent is drawn to the rise of the emission spectrum on the short-wavelength side, and the wavelength value λedge [nm] of the point of intersection of the tangent and the horizontal axis is substituted into the following equation (i) to calculate S1.

$$S1\ [eV] = 1239.85/\lambda edge \qquad (i)$$

For T1, on the other hand, the phosphorescence spectrum of the above deposition film is measured, a tangent is drawn to the rise of the phosphorescence spectrum on the short-wavelength side, and the wavelength value λedge [nm] of the point of intersection of the tangent and the horizontal axis is substituted into the following equation (ii) to calculate T1.

$$T1\ [eV] = 1239.85/\lambda edge \qquad (ii)$$

The known host materials are known in a large number of Patent Literatures and the like, and hence may be selected from them. Specific examples of the host material include, but are not particularly limited to, various metal complexes typified by metal complexes of indole compounds, carbazole compounds, indolocarbazole compounds, pyridine compounds, pyrimidine compounds, triazine compounds, triazole compounds, oxazole compounds, oxadiazole compounds, imidazole compounds, phenylenediamine compounds, arylamine compounds, anthracene compounds, fluorenone compounds, stilbene compounds, triphenylene compounds, carborane compounds, porphyrin compounds, phthalocyanine compounds, and 8-quinolinol compounds, and metal phthalocyanine, and metal complexes of benzoxazole and benzothiazole compounds; and polymer compounds such as poly(N-vinyl carbazole) compounds, aniline-based copolymer compounds, thiophene oligomers, polythiophene compounds, polyphenylene compounds, polyphenylene vinylene compounds, and polyfluorene compounds. Preferred examples thereof include carbazole compounds, indolocarbazole compounds, pyridine compounds, pyrimidine compounds, triazine compounds, anthracene compounds, triphenylene compounds, carborane compounds, and porphyrin compounds.

A preferred known host is not particularly limited, but specific examples thereof include the following.

[C23]

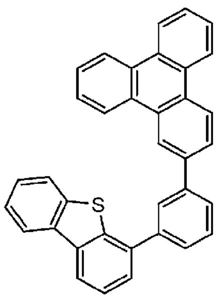

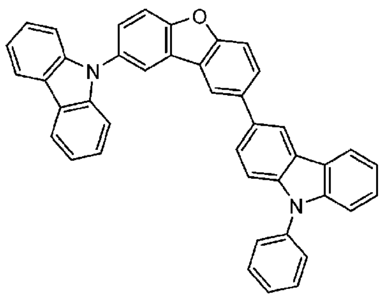

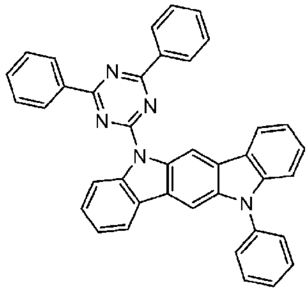

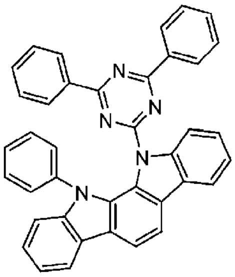

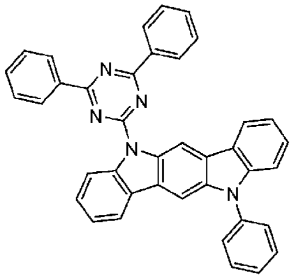

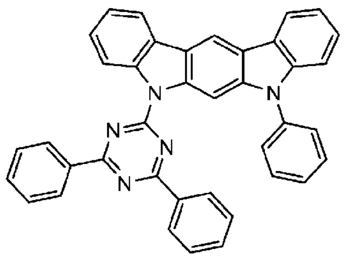

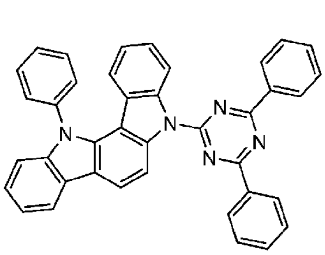

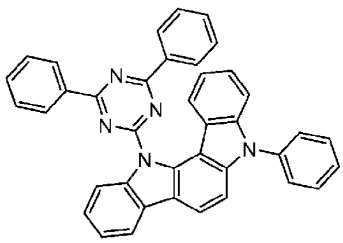

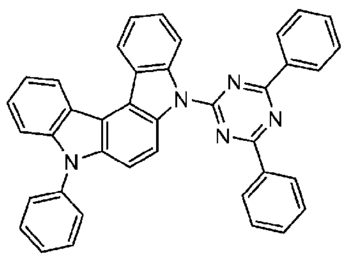

[C24]

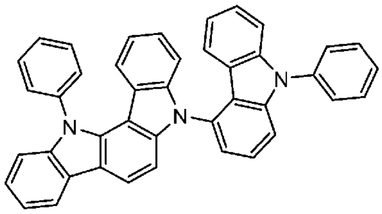

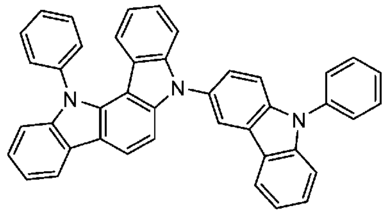

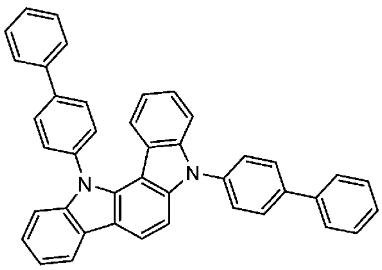

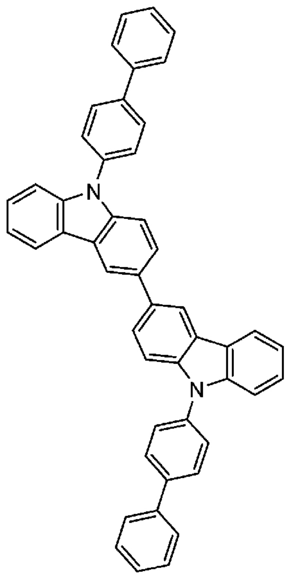

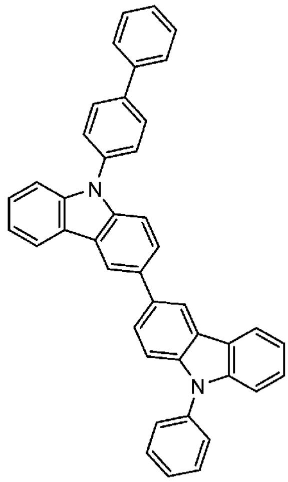

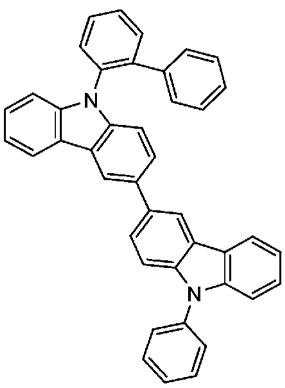

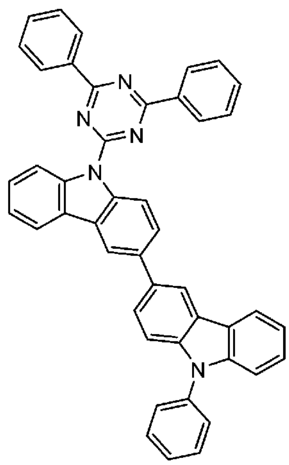

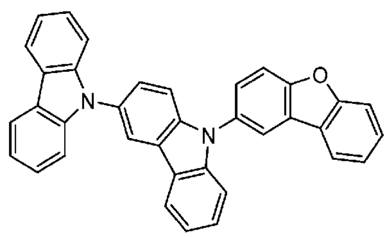

[C25]

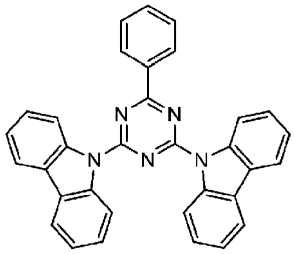

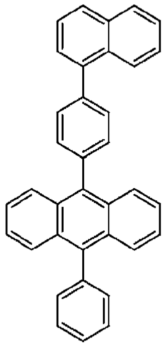

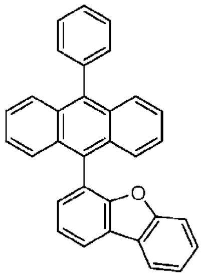

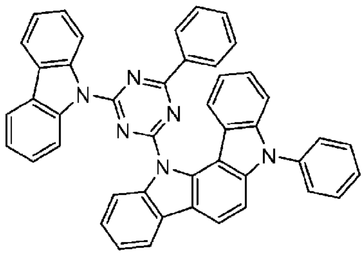

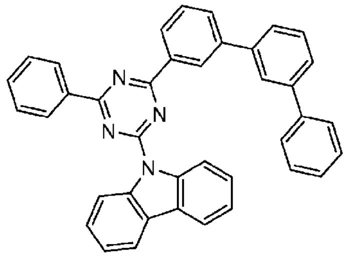

When a plurality of hosts is used, each host is deposited from different deposition sources, or a plurality of hosts is premixed before vapor deposition to form a premix, whereby a plurality of hosts can be simultaneously deposited from one deposition source.

When a plurality of hosts is used, the host is preferably the mixed composition for an organic electroluminescent device obtained by mixing the compound represented by the general formula (1) and the cyclic azine compound.

When two types of hosts are premixed and then used, the difference in 50% weight reduction temperature ($T_{50}$) is desirably small to produce an organic EL device having good characteristics with good reproducibility. The 50% weight reduction temperature refers to a temperature at which the weight is reduced by 50%, when the temperature is raised from room temperature at a rate of 10°C per minute until 550°C in TG-DTA measurement under reduced nitrogen gas pressure (1 Pa). The vaporization due to evaporation or sublimation is considered to be most frequently generated around this temperature.

The difference in the 50% weight reduction temperature between the two types of hosts in the premix is preferably within 20°C. By vaporizing and depositing the premix from a single deposition source, a uniform deposition film can be obtained. In this case, the light emitting dopant material that is necessary to form the light emitting layer or a further host used as needed may be mixed into the premix, but when there is a large difference in the temperatures at which they reach a desired vapor pressure, the light emitting dopant material and the further host are preferably deposited from other deposition sources.

As for the mixing ratio (weight ratio) of the first host to the second host when two types of hosts are used, the proportion of the first host is 40 to 90%, preferably 50 to 90%, and more preferably 60 to 90% based on the total amount of the first host and the second host. When the compound represented by the general formula (1) is used as the host, the first host is the compound represented by the general formula (1).

As the method of premixing, a method by which hosts can be mixed as uniformly as possible is desirable, and examples thereof include, but are not limited to, milling, a method of heating and melting hosts under reduced pressure or under an inert gas atmosphere such as nitrogen, and sublimation.

The host and a premix thereof may be in the form of powder, sticks, or granules.

-Injection Layer-

The injection layer refers to a layer provided between the electrode and the organic layer to reduce the driving voltage and improve the light emission brightness, and includes the hole injection layer and the electron injection layer. The injection layer may be present between the anode and the light emitting layer or the hole transport layer, as well as between the cathode and the light emitting layer or the electron transport layer. The injection layer may be provided as necessary.

-Hole Blocking Layer-

The hole blocking layer has the function of the electron transport layer in a broad sense, is made of a hole blocking material having a very small ability to transport holes while having the function of transporting electrons, and can improve the recombination probability between the electrons and the holes in the light emitting layer by blocking the holes while transporting the electrons. For the hole blocking layer, a known hole blocking material can be used. A plurality of hole blocking materials may be used in combination.

-Electron Blocking Layer-

The electron blocking layer has the function of the hole transport layer in a broad sense, and can improve the recombination probability between the electrons and the holes in the light emitting layer by blocking the electrons while transporting the holes. As the material for the electron blocking layer, the compound represented by the general formula (1) or the mixed composition is preferably used, and a known electron blocking layer material can be used. When the compound represented by the general formula (1) or the mixed composition is used for the electron blocking layer, the compound represented by the general formula (1), a known host material described above, and a host material obtained by combining a plurality of the host materials may be used as the host material.

The layer adjacent to the light emitting layer includes the hole blocking layer, and the electron blocking layer, and when these layers are not provided, the adjacent layer is the hole transport layer, the electron transport layer, and the like.

-Hole Transport Layer-

The hole transport layer is made of a hole transport material having the function of transporting holes, and the hole transport layer may be provided as a single layer or a plurality of layers.

The hole transport material has any of hole injection properties, hole transport properties, or electron barrier properties, and may be either an organic material or an inorganic material. As the hole transport material, any of conventionally known compounds may be selected and used. Examples of such a hole transport material include porphyrin derivatives, arylamine derivatives, triazole derivatives, oxadiazole derivatives, imidazole derivatives, polyarylalkane derivatives, phenylenediamine derivatives, arylamine derivatives, amino-substituted chalcone derivatives, oxazole derivatives, styrylanthracene derivatives, fluorenone derivatives, hydrazone derivatives, stilbene derivatives, silazane derivatives, aniline-based copolymers, and conductive polymer oligomers, particularly, thiophene oligomers.

-Electron Transport Layer-

The electron transport layer is made of a material having the function of transporting electrons, and the electron transport layer may be provided as a single layer or a plurality of layers.

The electron transport material (may also serve as the hole blocking material) has the function of transmitting electrons injected from the cathode to the light emitting layer. As the electron transport layer, any of conventionally known compounds may be selected and used, and examples thereof include polycyclic aromatic derivatives such as naphthalene, anthracene, and phenanthroline, tris(8-quinolinolato)aluminum (III) derivatives, phosphine oxide derivatives, nitrosubstituted fluorene derivatives, diphenylquinone derivatives, thiopyran dioxide derivatives, carbodiimides, fluorenylidene methane derivatives, anthraquinodimethane and anthrone derivatives, bipyridine derivatives, quinoline derivatives, oxadiazole derivatives, benzimidazole derivatives, benzothiazole derivatives, and indolocarbazole derivatives. Further, polymer materials in which these materials are introduced in the polymer chain or these materials constitute the main chain of the polymer can also be used.

When the organic EL device of the present invention is produced, the film formation method of each layer is not particularly limited, and the layers may be produced by either a dry process or a wet process.

Examples

Hereinafter, the present invention will be described in further detail with reference to Examples, but the present invention is not limited to these Examples.

The compounds used in Examples and Comparative Examples are shown below.

[C26]

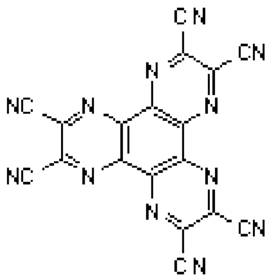

HAT-CN

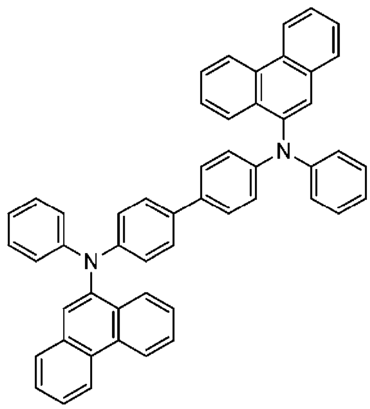

HT-1

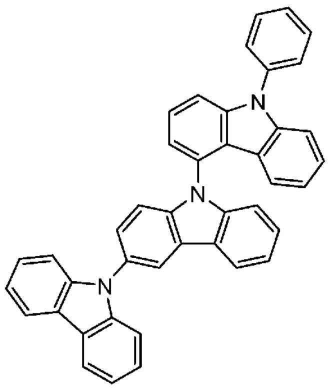

HT-2

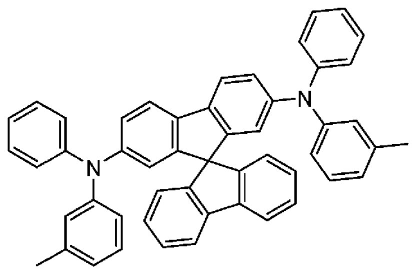

HT-3

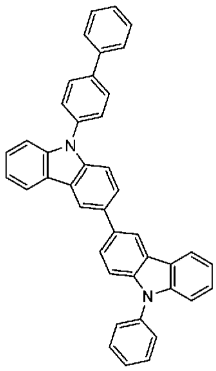

HT-4

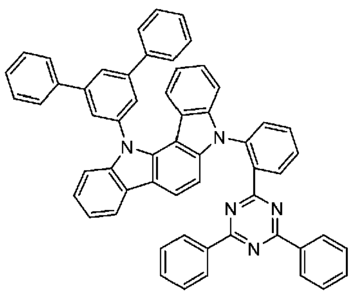

BH-1(2-11)

[C27]

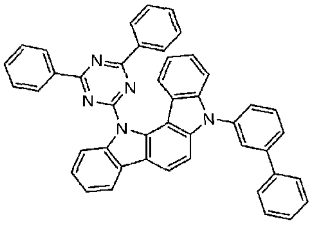
BH-2(2-12)

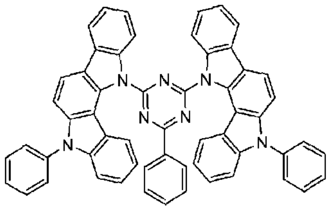
BH-3(2-15)

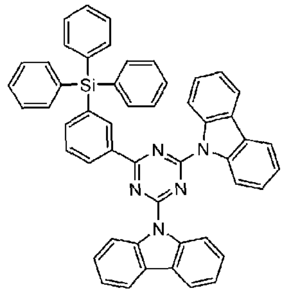
BH-4 (2-22)

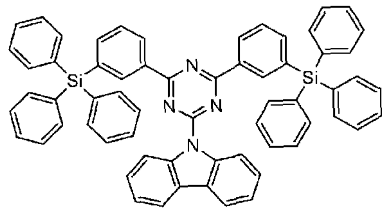
BH-5 (2-31)

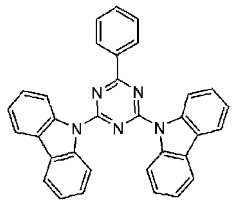
BH-6 (2-20)

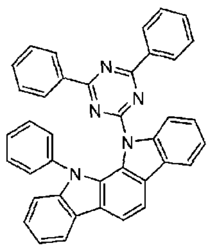
GH-1(2-1)

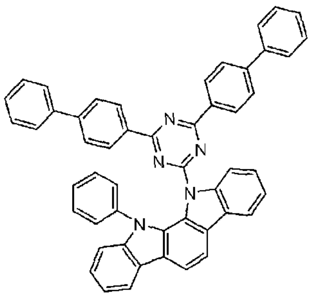
GH-2(2-26)

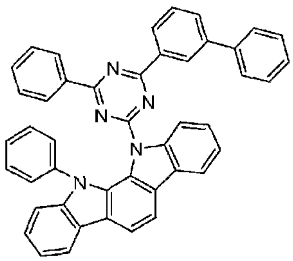
GH-3(2-2)

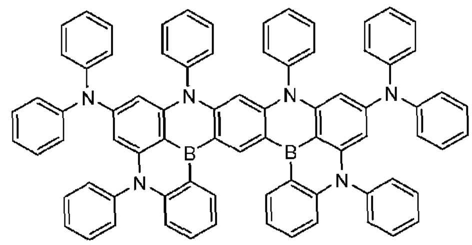
BD-1

[C28]

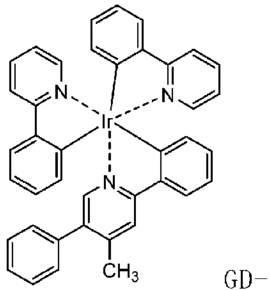

GD-1

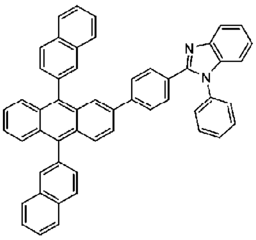

ET-1

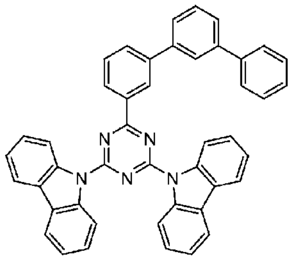

ET-2

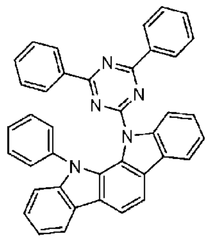

ET-3

Synthesis Example 1

[C29]

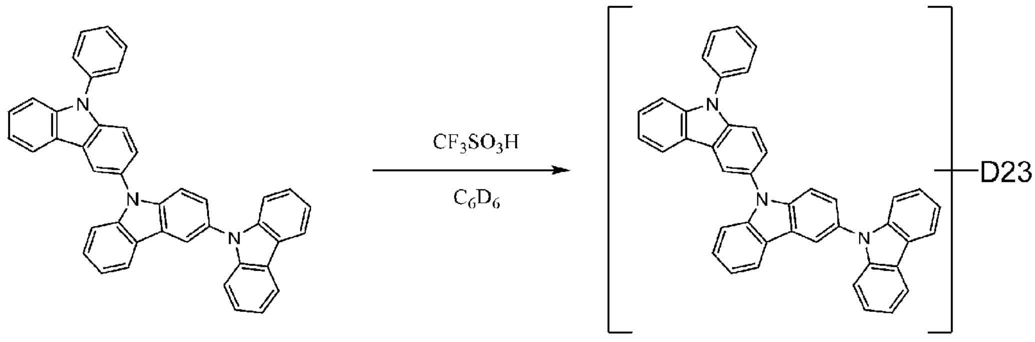


(A)

1-15-D23

As shown in the above reaction scheme, 1.0 g of a raw material (A), 1.3 g of trifluoromethanesulfonic acid, and 9.9 g of deuterated benzene were put in a three-necked flask under a nitrogen atmosphere and stirred at 50°C for 3 hours. After the reaction solution was cooled to room temperature, a solution obtained by dissolving 1.0 g of sodium carbonate in 9.9 g of heavy water was put in the reaction solution and stirred for 1 hour. The reaction solution was washed with water, and then concentrated. The concentrate was purified by silica gel column chromatography and recrystallization, and thereafter the resulting solid was dried to yield 0.4 g of a compound (1-15-D23) (yield: 39%).
APCI-TOFMS m/z 598 $[M+1]^+$

Synthesis Example 2

[C30]

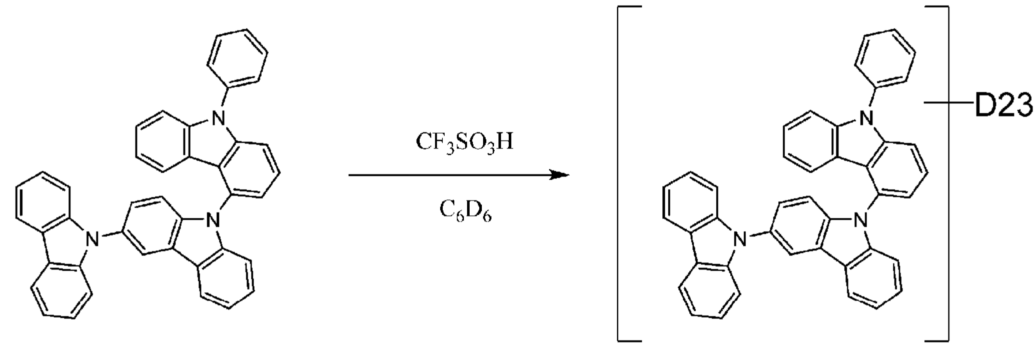


(B)

1-19-D23

As shown in the above reaction scheme, 1.0 g of a raw material (B), 1.3 g of trifluoromethanesulfonic acid, and 9.9 g of deuterated benzene were put in a three-necked flask under a nitrogen atmosphere and stirred at 50°C for 3 hours. After the reaction solution was cooled to room temperature, a solution obtained by dissolving 1.0 g of sodium carbonate in 9.9 g of heavy water was put in the reaction solution and stirred for 1 hour. The reaction solution was washed with water, and then concentrated. The concentrate was purified by silica gel column chromatography and recrystallization, and thereafter the resulting solid was dried to yield 0.3 g of a compound (1-19-D23) (yield: 29%).
APCI-TOFMS m/z 598 $[M+1]^+$

Synthesis Example 3

[C31]

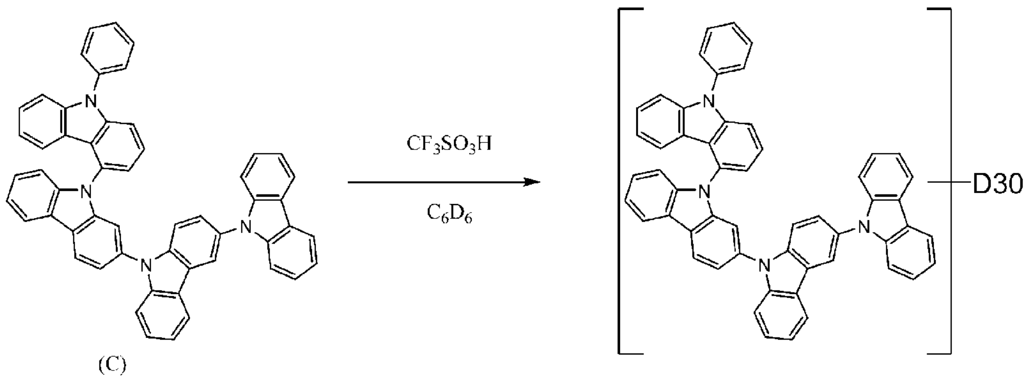


(C)

1-83-D30

As shown in the above reaction scheme, 1.0 g of a raw material (C), 1.0 g of trifluoromethanesulfonic acid, and 9.7 g of deuterated benzene were put in a three-necked flask under a nitrogen atmosphere and stirred at 50°C for 3 hours. After the reaction solution was cooled to room temperature, a solution obtained by dissolving 0.8 g of sodium carbonate in 9.7 g of heavy water was put in the reaction solution and stirred for 1 hour. The reaction solution was washed with water, and then concentrated. The concentrate was purified by silica gel column chromatography and recrystallization, and thereafter the resulting solid was dried to yield 0.2 g of a compound (1-83-D30) (yield: 19%).
APCI-TOFMS m/z 770 $[M+1]^+$

Synthesis Example 4

[C32]

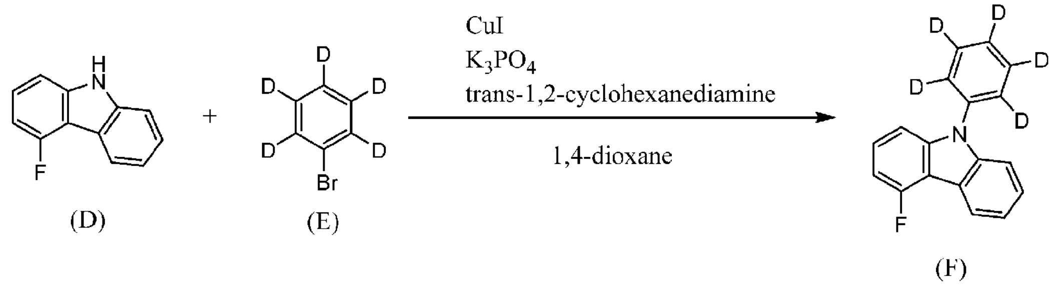


(D) (E) (F)

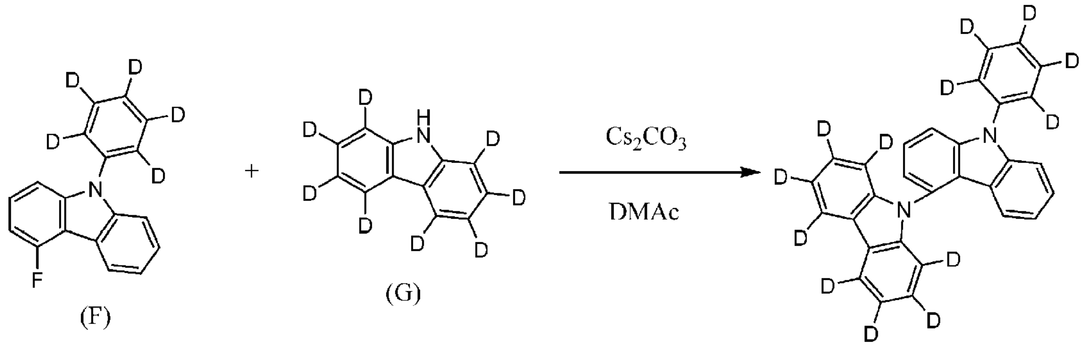


(F) (G)

1-4-D13

As shown in the above reaction scheme, under a nitrogen atmosphere, 3.0 g of a raw material (D), 2.9 g of a raw material (E), 0.2 g of copper(I) iodide, 17.2 g of tripotassium phosphate, and 100 ml of 1,4-dioxane were put in a three-necked flask and stirred at room temperature for 30 minutes. Thereafter, 0.2 g of trans-1,2-cyclohexane diamine was put and stirred at 120°C for 72 hours. The reaction solution was cooled to room temperature, and then filtered, and the filtrate was concentrated. The concentrate was purified by silica gel column chromatography and recrystallization, and thereafter the resulting solid was dried to yield 3.9 g of an intermediate raw material (F) (yield: 91%).

As shown in the above reaction scheme, 3.0 g of the intermediate raw material (F), 2.2 g of a raw material (G), 18.4 g of cesium carbonate, and 100 ml of N,N-dimethylacetamide were put in a three-necked flask under a nitrogen atmosphere and stirred at 180°C for 10 hours. The reaction solution was cooled to room temperature, and then filtered, the filtrate was put in a flask containing water (500 ml) and stirred, and the precipitated solid was collected by filtering. The solid collected by filtering was dissolved in tetrahydrofuran (200 ml) and extracted with toluene (200 ml). The extracted solution was concentrated. The concentrate was purified by silica gel column chromatography and recrystallization, and thereafter the resulting solid was dried to yield 3.0 g of a compound (1-4-D13) (yield: 62%) .

APCI-TOFMS m/z 423 $[M+1]^+$

Synthesis Example 5

[C33]

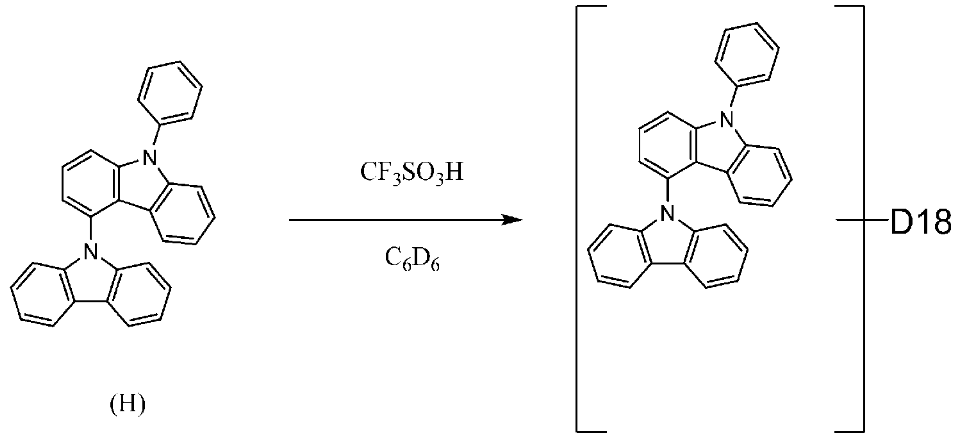


1-4-D18

As shown in the above reaction scheme, 1.0 g of a raw material (H), 1.8 g of trifluoromethanesulfonic acid, and 10.3 g of deuterated benzene were put in a three-necked flask under a nitrogen atmosphere and stirred at 50°C for 3 hours. After the reaction solution was cooled to room temperature, a solution obtained by dissolving 1.4 g of sodium carbonate in 10.3 g of heavy water was put in the reaction solution and stirred for 1 hour. The reaction solution was washed with water, and then concentrated. The concentrate was purified by silica gel column chromatography and recrystallization, and thereafter the resulting solid was dried to yield 0.5 g of a compound (1-4-D18) (yield: 48%).
APCI-TOFMS m/z 428 $[M+1]^+$

Synthesis Example 6

[C34]

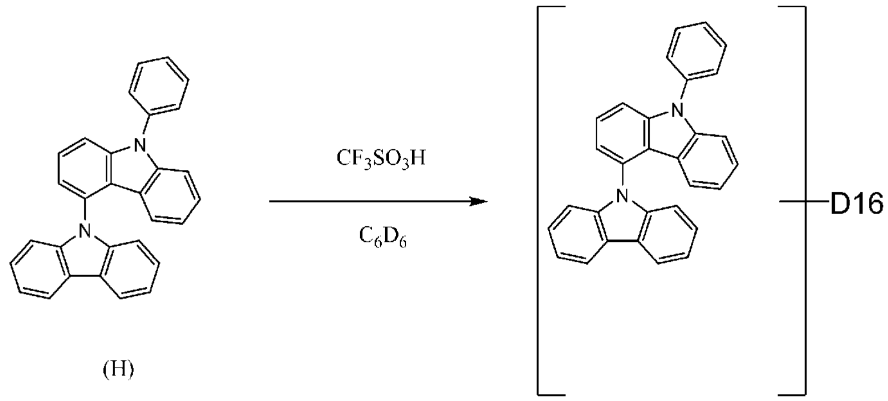


1-4-D16

As shown in the above reaction scheme, 1.0 g of a raw material (H), 1.8 g of trifluoromethanesulfonic acid, and 10.3 g of deuterated benzene were put in a threenecked flask under a nitrogen atmosphere and stirred at 50°C for 2.5 hours. After the reaction solution was cooled to room temperature, a solution obtained by dissolving 1.4 g of sodium carbonate in 10.3 g of heavy water was put in the reaction solution and stirred for 1 hour. The reaction solution was washed with water, and then concentrated. The concentrate was purified by silica gel column chromatography and recrystallization, and thereafter the resulting solid was dried to yield 0.5 g of a compound (1-4-D16) (yield: 48%).
APCI-TOFMS m/z 426 $[M+1]^+$

Synthesis Example 7

[C35]

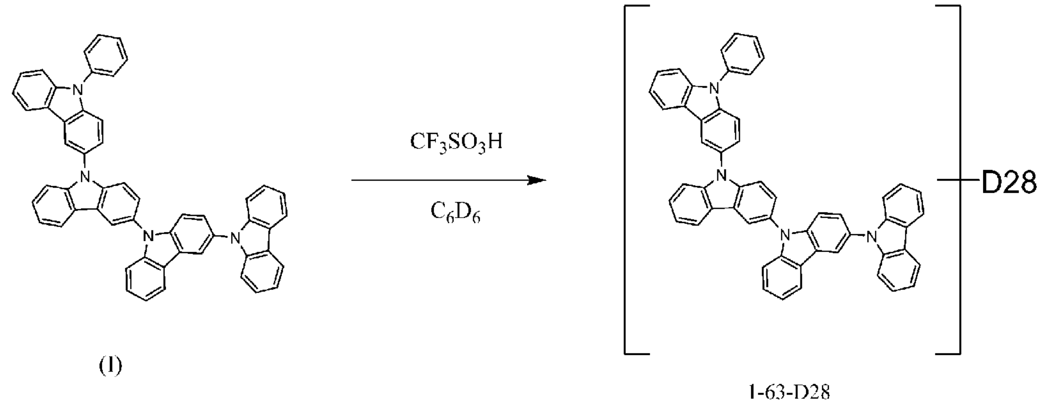


(I)

1-63-D28

As shown in the above reaction scheme, 1.0 g of raw material (I), 1.0 g of trifluoromethanesulfonic acid, and 9.7 g of deuterated benzene were put in a three-necked flask under a nitrogen atmosphere and stirred at 50°C for 2.5 hours. After the reaction solution was cooled to room temperature, a solution obtained by dissolving 0.8 g of sodium carbonate in 9.7 g of heavy water was put in the reaction solution and stirred for 1 hour. The reaction solution was washed with water, and then concentrated. The concentrate was purified by silica gel column chromatography and crystallization, and thereafter the resulting solid was dried to yield 0.3 g of a compound (1-63-D28) (yield: 29%).
APCI-TOFMS m/z 768 $[M+1]^+$

Synthesis Example 8

[C36]

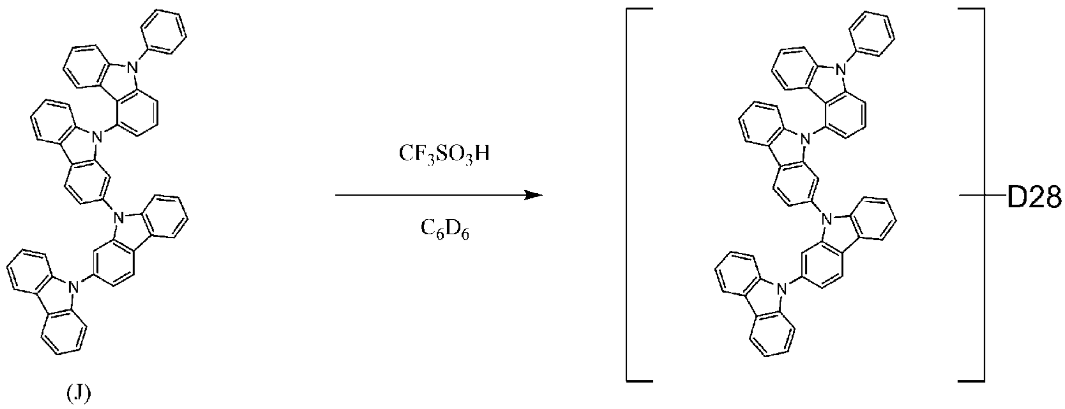


(J)

1-74-D28

As shown in the above reaction scheme, 1.0 g of a raw material (J), 1.0 g of trifluoromethanesulfonic acid, 9.7 g of deuterated benzene were put in a three-necked flask under a nitrogen atmosphere and stirred at 50°C for 3.5 hours. After the reaction solution was cooled to room temperature, a solution obtained by dissolving 0.8 g of sodium carbonate in 9.7 g of heavy water was put in the reaction solution and stirred for 1 hour. The reaction solution was washed with water, and then concentrated. The concentrate was purified by silica gel column chromatography and crystallization, and thereafter the resulting solid was dried to yield 0.3 g of a compound (1-74-D28) (yield: 29%).
APCI-TOFMS m/z 768 $[M+1]^+$

Synthesis Example 9

[C37]

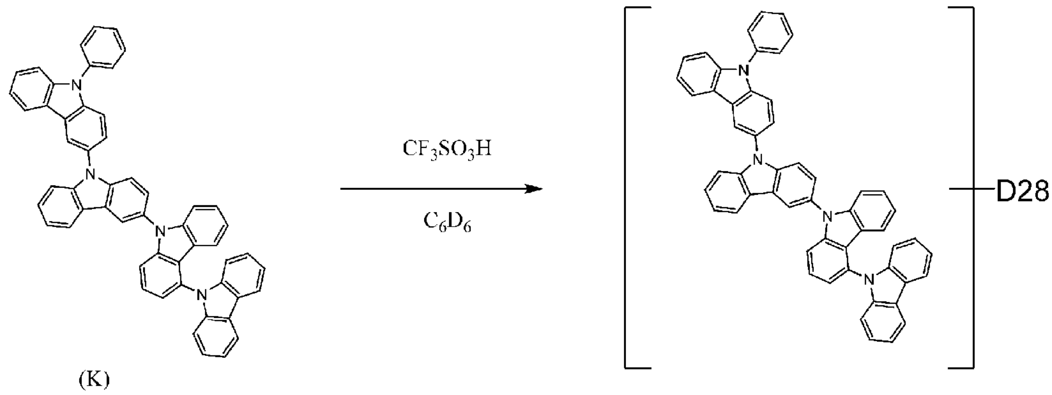


(K)

1-64-D28

As shown in the above reaction scheme, 1.0 g of a raw material (K), 1.0 g of trifluoromethanesulfonic acid, and 9.7 g of deuterated benzene were put in a three-necked flask under a nitrogen atmosphere and stirred at 50°C for 3 hours. After the reaction solution was cooled to room temperature, a solution obtained by dissolving 0.8 g of sodium carbonate in 9.7 g of heavy water was put in the reaction solution and stirred for 1 hour. The reaction solution was washed with water, and then concentrated. The concentrate was purified by silica gel column chromatography and crystallization, and thereafter the resulting solid was dried to yield 0.3 g of a compound (1-64-D28) (yield: 29%).
APCI-TOFMS m/z 768 $[M+1]^+$

Synthesis Example 10

[C38]

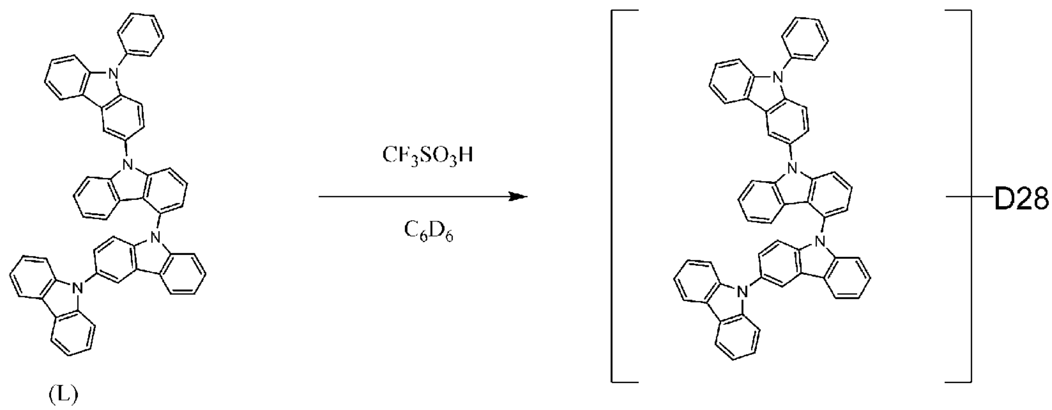


(L)

1-67-D28

As shown in the above reaction scheme, 1.0 g of a raw material (L), 1.0 g of trifluoromethanesulfonic acid, and 9.7 g of deuterated benzene were put in a three-necked flask under a nitrogen atmosphere and stirred at 50°C for 3.5 hours. After the reaction solution was cooled to room temperature, a solution obtained by dissolving 0.8 g of sodium carbonate in 9.7 g of heavy water was put in the reaction solution and stirred for 1 hour. The reaction solution was washed with water, and then concentrated. The concentrate was purified by silica gel column chromatography and crystallization, and thereafter the resulting solid was dried to yield 0.3 g of a compound (1-67-D28) (yield: 29%).
APCI-TOFMS m/z 768 $[M+1]^+$

Synthesis Example 11

[C39]

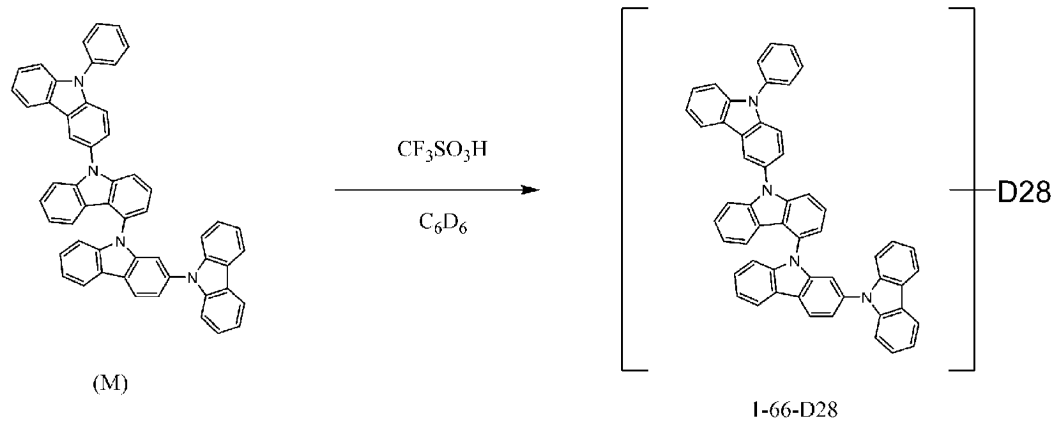


(M)

1-66-D28

As shown in the above reaction scheme, 1.0 g of a raw material (M), 1.0 g of trifluoromethanesulfonic acid, and 9.7 g of deuterated benzene were put in a three-necked flask under a nitrogen atmosphere and stirred at 50°C for 3.5 hours. After the reaction solution was cooled to room temperature, a solution obtained by dissolving 0.8 g of sodium carbonate in 9.7 g of heavy water was put in the reaction solution and stirred for 1 hour. The reaction solution was washed with water, and then concentrated. The concentrate was purified by silica gel column chromatography and crystallization, and thereafter the resulting solid was dried to yield 0.3 g of a compound (1-66-D28) (yield: 29%).
APCI-TOFMS m/z 768 $[M+1]^+$

Synthesis Example 12

[C40]

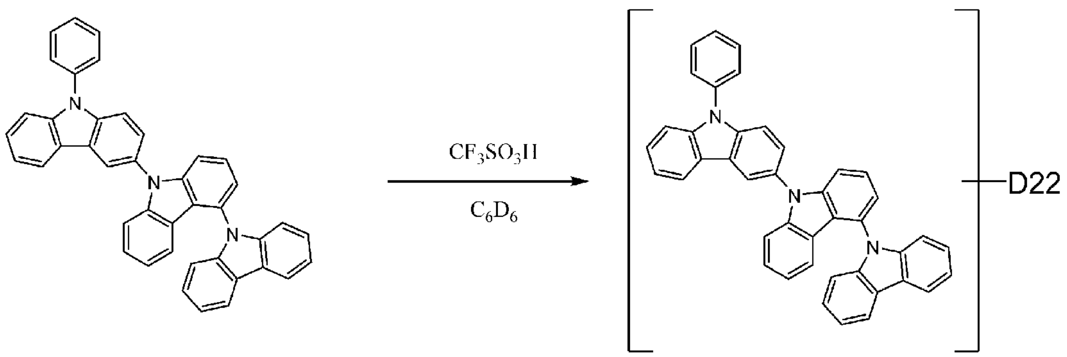


(O)

1-16-D22

As shown in the above reaction scheme, 1.0 g of a raw material (O), 1.3 g of trifluoromethanesulfonic acid, and 9.9 g of deuterated benzene were put in a three-necked flask under a nitrogen atmosphere and stirred at 50°C for 2.5 hours. After the reaction solution was cooled to room temperature, a solution obtained by dissolving 1.0 g of sodium carbonate in 9.9 g of heavy water was put in the reaction solution and stirred for 1 hour. The reaction solution was washed with water, and then concentrated. The concentrate was purified by silica gel column chromatography and recrystallization, and thereafter the resulting solid was dried to yield 0.4 g of a compound (1-16-D22) (yield: 39%).
APCI-TOFMS m/z 597 $[M+1]^+$

Synthesis Example 13

[C41]

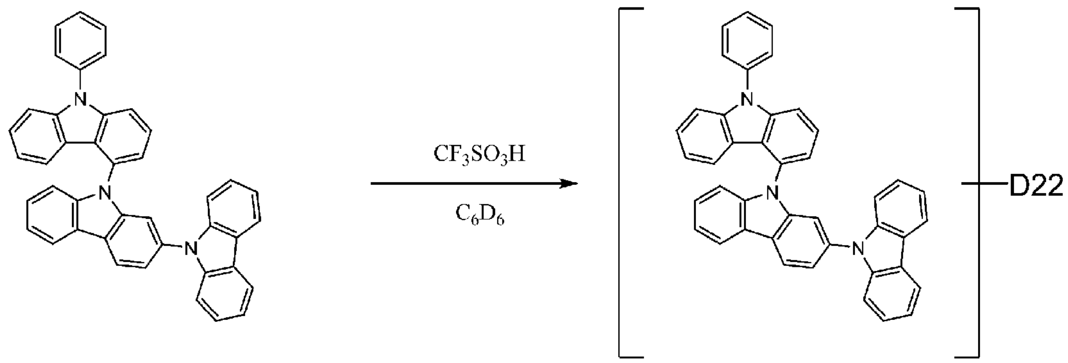


(P)

1-18-D22

As shown in the above reaction scheme, 1.0 g of a raw material (P), 1.3 g of trifluoromethanesulfonic acid, and 9.9 g of deuterated benzene were put in a three-necked flask under a nitrogen atmosphere and stirred at 50°C for 2.5 hours. After the reaction solution was cooled to room temperature, a solution obtained by dissolving 1.0 g of sodium carbonate in 9.9 g of heavy water was put in the reaction solution and stirred for 1 hour. The reaction solution was washed with water, and then concentrated. The concentrate was purified by silica gel column chromatography and recrystallization, and thereafter the resulting solid was dried to yield 0.4 g of a compound (1-18-D22) (yield: 39%).
APCI-TOFMS m/z 597 $[M+1]^+$

For each compound obtained in the above Synthesis Examples 1 to 13, the deuteration ratio was determined by mass spectrometry. In the mass spectrometry, a molecular ion peak value having the highest intensity was measured as the main molecular weight of the deuterated compound. The molecular weight of a non-deuterated compound corresponding to the deuterated compound was measured in the same manner, and the difference between the molecular weight of the deuterated compound and the molecular weight of the non-deuterated compound was determined, thereby calculating an average value of the number of replaced deuterium of the deuterated compound per molecule. The deuteration ratio was determined by calculating the proportion of the total number of deuterium to the total number of deuterium and hydrogen in the deuterated compound. In addition, Comparative Compounds 1 to 5 shown below were synthesized, and the deuteration ratios thereof were determined by mass spectrometry in the same manner as in the case of the deuterated compounds according to Synthesis Examples 1 to 13. These results are collectively shown in Table 1.

[C42]

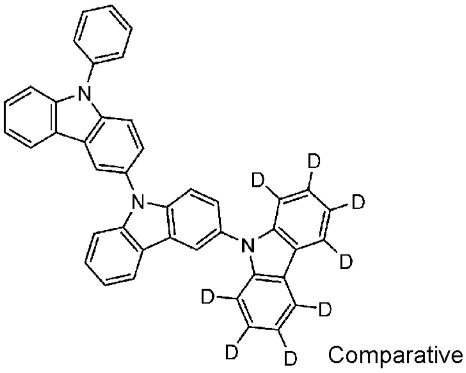
Comparative Example 1

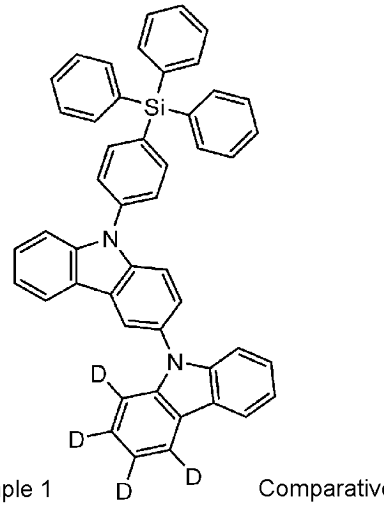
Comparative Example 2

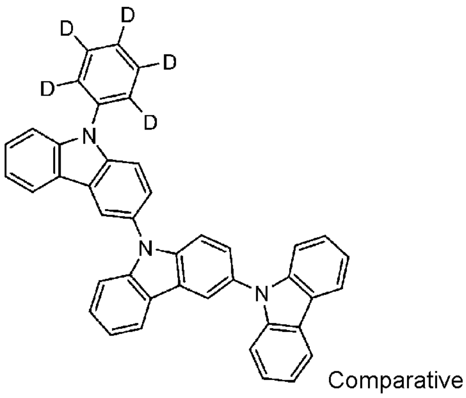
Comparative Example 3

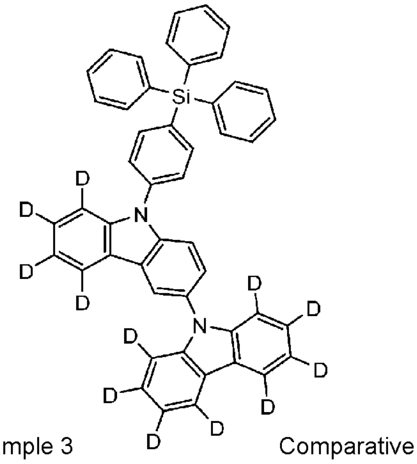
Comparative Example 4

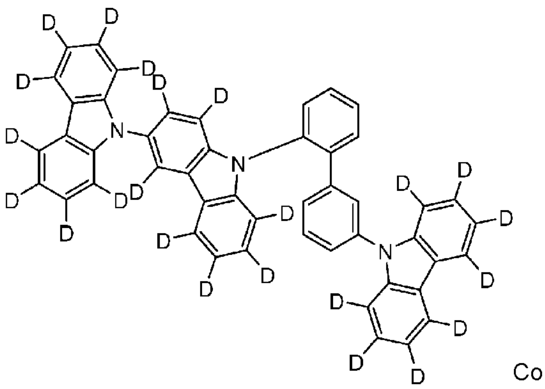
Comparative Example 5

[Table 1]

| Compound name | Number of deuterium | Total number of hydrogen and deuterium | Deuteration ratio |
|---|---|---|---|
| 1-15-D23 | 23 | 27 | 85% |
| 1-19-D23 | 23 | 27 | 85% |
| 1-83-D30 | 30 | 34 | 88% |
| 1-4-D13 | 13 | 20 | 65% |

(continued)

| Compound name | Number of deuterium | Total number of hydrogen and deuterium | Deuteration ratio |
|---|---|---|---|
| 1-4-D18 | 18 | 20 | 90% |
| 1-4-D16 | 16 | 20 | 80% |
| 1-63-D28 | 28 | 34 | 82% |
| 1-74-D28 | 28 | 34 | 82% |
| 1-64-D28 | 28 | 34 | 82% |
| 1-67-D28 | 28 | 34 | 82% |
| 1-66-D28 | 28 | 34 | 82% |
| 1-16-D22 | 22 | 27 | 81% |
| 1-18-D22 | 22 | 27 | 81% |
| Comparative Compound 1 | 8 | 27 | 30% |
| Comparative Compound 2 | 4 | 34 | 12% |
| Comparative Compound 3 | 5 | 27 | 19% |
| Comparative Compound 4 | 12 | 34 | 35% |
| Comparative Compound 5 | 23 | 31 | 74% |

Example 1

Each thin film shown below was laminated on the glass substrate on which an anode made of ITO having a film thickness of 70 nm was formed by a vacuum deposition method at a degree of vacuum of $4.0 \times 10^{-5}$ Pa. First, the previously presented HAT-CN was formed on ITO to a thickness of 10 nm as a hole injection layer, and then HT-1 was formed to a thickness of 25 nm as a hole transport layer. Then, HT-2 was formed to a thickness of 5 nm as an electron blocking layer. Then, the compound (1-19-D23) as the host and BD-1 as the thermally activated delayed fluorescence dopant were co-deposited from different deposition sources to form a light emitting layer having a thickness of 30 nm. At this time, they were co-deposited under deposition conditions such that the concentration of BD-1 was 2 wt%. Then, ET-2 was formed to a thickness of 5 nm as a hole blocking layer. Then, ET-1 was formed to a thickness of 40 nm as an electron transport layer. Further, lithium fluoride (LiF) was formed on the electron transport layer to a thickness of 1 nm as an electron injection layer. Finally, aluminum (Al) was formed on the electron injection layer to a thickness of 70 nm as a cathode, whereby an organic EL device according to Example 1 was produced.

Comparative Example 1

Each organic EL device was produced in the same manner as in Example 1, except that HT-2 was used as the host.

Example 2

Each thin film shown below was laminated on the glass substrate on which an anode made of ITO having a film thickness of 70 nm was formed by a vacuum deposition method at a degree of vacuum of $4.0 \times 10^{-5}$ Pa. First, the previously presented HAT-CN was formed on ITO to a thickness of 10 nm as a hole injection layer, and then HT-1 was formed to a thickness of 25 nm as a hole transport layer. Then, HT-2 was formed to a thickness of 5 nm as an electron blocking layer. Then, the compound (1-19-D23) as the first host, BH-1 as a second host, and BD-1 as the thermally activated delayed fluorescence dopant were co-deposited from different deposition sources to form a light emitting layer having a thickness of 30 nm. At this time, they were co-deposited under deposition conditions such that the concentration of BD-1 was 1 wt% and the weight ratio of the first host to the second host was 70:30. Then, ET-2 was formed to a thickness of 5 nm as a hole blocking layer. Then, ET-1 was formed to a thickness of 40 nm as an electron transport layer. Further, lithium fluoride (LiF) was formed on the electron transport layer to a thickness of 1 nm as an electron injection layer. Finally, aluminum (Al) was formed on the electron injection layer to a thickness of 70 nm as a cathode, whereby an organic EL device according to Example 1 was produced.

Examples 3 to 19 and Comparative Examples 2 to 9, A to C

Each organic EL device was produced in the same manner as in Example 2, except that the electron blocking layer material, the first host, and the second host were changed to the compounds shown in Table 2.

[Table 2]

| | Electron blocking layer | First host | Second host |
|---|---|---|---|
| Example 1 | HT-2 | 1-19-D23 | - |
| Example 2 | HT-2 | 1-15-D23 | BH-1 |
| Example 3 | HT-2 | 1-19-D23 | BH-1 |
| Example 4 | HT-2 | 1-83-D30 | BH-1 |
| Example 5 | HT-2 | 1-4-D13 | BH-1 |
| Example 6 | HT-2 | 1-4-D18 | BH-1 |
| Example 7 | HT-2 | 1-19-D23 | BH-2 |
| Example 8 | HT-2 | 1-19-D23 | BH-3 |
| Example 9 | 1-19-D23 | 1-19-D23 | BH-1 |
| Example 10 | HT-2 | 1-19-D23 | BH-4 |
| Example 11 | HT-2 | 1-19-D23 | BH-5 |
| Example 12 | HT-2 | 1-4-D16 | BH-6 |
| Example 13 | HT-2 | 1-63-D28 | BH-6 |
| Example 14 | HT-2 | 1-74-D28 | BH-6 |
| Example 15 | HT-2 | 1-64-D28 | BH-6 |
| Example 16 | HT-2 | 1-67-D28 | BH-6 |
| Example 17 | HT-2 | 1-66-D28 | BH-6 |
| Example 18 | HT-2 | 1-16-D22 | BH-6 |
| Example 19 | HT-2 | 1-18-D22 | BH-6 |
| Comparative Example 1 | HT-2 | HT-2 | - |
| Comparative Example 2 | HT-2 | HT-2 | BH-1 |
| Comparative Example 3 | HT-2 | Comparative Compound 1 | BH-1 |
| Comparative Example 4 | HT-2 | Comparative Compound 2 | BH-1 |
| Comparative Example 5 | HT-2 | Comparative Compound 3 | BH-1 |
| Comparative Example 6 | HT-2 | Comparative Compound 4 | BH-1 |
| Comparative Example 7 | HT-2 | Comparative Compound 5 | BH-1 |
| Comparative Example 8 | HT-2 | Comparative Compound 2 | BH-4 |
| Comparative Example 9 | HT-2 | Comparative Compound 2 | BH-5 |
| Comparative Example A | HT-2 | HT-2 | BH-6 |
| Comparative Example B | HT-2 | Comparative Compound 1 | BH-6 |
| Comparative Example C | HT-2 | Comparative Compound 5 | BH-6 |

The emission color, voltage, power efficiency, and lifetime of each organic EL device produced in Examples and Comparative Examples are shown in Table 3. The emission color, voltage, and power efficiency are values at a current density of 2.5 $mA/cm^2$ and were initial characteristics. The time taken for the luminance to reduce to 70% of the initial luminance when the current density was 2.5 $mA/cm^2$ was measured as the lifetime.

[Table 3]

| | Emission color | Voltage (V) | Power efficiency (lm/W) | Lifetime (h) |
|---|---|---|---|---|
| Example 1 | Blue | 4.0 | 15.7 | 31 |
| Example 2 | Blue | 3.9 | 31.1 | 377 |
| Example 3 | Blue | 3.9 | 34.1 | 420 |
| Example 4 | Blue | 3.8 | 34.5 | 401 |
| Example 5 | Blue | 4.2 | 28.7 | 305 |
| Example 6 | Blue | 4.1 | 30.3 | 426 |
| Example 7 | Blue | 3.7 | 36.2 | 448 |
| Example 8 | Blue | 3.6 | 34.4 | 452 |
| Example 9 | Blue | 3.9 | 34.6 | 433 |
| Example 10 | Blue | 4.3 | 26.5 | 188 |
| Example 11 | Blue | 4.2 | 27.7 | 205 |
| Example 12 | Blue | 4.3 | 33.3 | 380 |
| Example 13 | Blue | 3.9 | 32.2 | 401 |
| Example 14 | Blue | 3.9 | 33.5 | 491 |
| Example 15 | Blue | 4.0 | 35.4 | 470 |
| Example 16 | Blue | 3.9 | 36.2 | 472 |
| Example 17 | Blue | 3.8 | 34.0 | 480 |
| Example 18 | Blue | 4.0 | 33.8 | 435 |
| Example 19 | Blue | 4.1 | 34.5 | 428 |
| Comparative Example 1 | Blue | 4.1 | 15.3 | 13 |
| Comparative Example 2 | Blue | 4.1 | 30.5 | 282 |
| Comparative Example 3 | Blue | 3.9 | 30.9 | 301 |
| Comparative Example 4 | Blue | 4.3 | 28.8 | 238 |
| Comparative Example 5 | Blue | 4.1 | 30.0 | 293 |
| Comparative Example 6 | Blue | 4.3 | 29.0 | 244 |
| Comparative Example 7 | Blue | 4.2 | 27.9 | 271 |
| Comparative Example 8 | Blue | 4.5 | 25.5 | 191 |
| Comparative Example 9 | Blue | 4.4 | 26.7 | 202 |
| Comparative Example A | Blue | 4.3 | 30.8 | 301 |
| Comparative Example B | Blue | 3.9 | 29.9 | 311 |
| Comparative Example C | Blue | 4.1 | 28.9 | 280 |

It is found from Examples and Comparative Examples of Table 3 that organic EL devices using the material for an organic electroluminescent device of the present invention as the electron blocking layer or the host of the organic EL device containing a thermally activated delayed fluorescence material in the light emitting layer exhibit blue light emission and have low voltage, efficiency, and long lifetime characteristics.

Example 20

Each thin film was laminated on the glass substrate on which an anode made of ITO having a film thickness of 110 nm was formed by a vacuum deposition method at a degree of vacuum of $4.0 \times 10^{-5}$ Pa. First, HAT-CN was formed on ITO to a thickness of 25 nm as a hole injection layer, and then HT-3 was formed to a thickness of 30 nm as a hole transport layer.

Then, HT-4 was formed to a thickness of 10 nm as an electron blocking layer. Then, the compound (1-15-D23) as the first host, GH-1 as the second host, and GD-1 as the phosphorescence dopant were co-deposited from different deposition sources to form a light emitting layer having a thickness of 40 nm. At this time, they were co-deposited under deposition conditions such that the concentration of GD-1 was 5 wt% and the weight ratio of the first host to the second host was 50:50. Then, ET-1 was formed to a thickness of 20 nm as an electron transport layer. Further, lithium fluoride (LiF) was formed on the electron transport layer to a thickness of 1 nm as an electron injection layer. Finally, aluminum (Al) was formed on the electron injection layer to a thickness of 70 nm as a cathode, whereby an organic EL device was produced.

Examples 21 to 25 and Comparative Examples 10 to 11

Each organic EL device was produced in the same manner as in Example 20, except that the first host, and the second host were changed to the compounds shown in Table 4.

[Table 4]

| | First host | Second host |
|---|---|---|
| Example 20 | 1-15-D23 | GH-1 |
| Example 21 | 1-15-D23 | GH-2 |
| Example 22 | 1-19-D23 | GH-1 |
| Example 23 | 1-19-D23 | GH-2 |
| Example 24 | 1-83-D30 | GH-1 |
| Example 25 | 1-83-D30 | GH-2 |
| Comparative Example 10 | HT-2 | GH-1 |
| Comparative Example 11 | HT-2 | GH-2 |

The emission color, voltage, power efficiency, and lifetime of each organic EL device produced in Examples and Comparative Examples are shown in Table 5. The emission color, voltage, and power efficiency are values at a current density of 20 $mA/cm^2$ and were initial characteristics. The time taken for the luminance to reduce to 95% of the initial luminance when the current density was 20 $mA/cm^2$ was measured as the lifetime.

[Table 5]

| | Emission color | Voltage (V) | Power efficiency (lm/W) | Lifetime (h) |
|---|---|---|---|---|
| Example 20 | Green | 3.9 | 48.2 | 101 |
| Example 21 | Green | 3.6 | 45.4 | 122 |
| Example 22 | Green | 4.0 | 48.8 | 100 |
| Example 23 | Green | 3.7 | 46.0 | 122 |
| Example 24 | Green | 3.8 | 49.1 | 109 |
| Example 25 | Green | 3.6 | 48.1 | 126 |
| Comparative Example 10 | Green | 3.9 | 47.5 | 81 |
| Comparative Example 11 | Green | 3.7 | 45.2 | 103 |

It is found from Examples and Comparative Examples of Table 5 that organic EL devices using the material for an organic electroluminescent device of the present invention as the host of the organic EL device containing a phosphorescence material in the light emitting layer exhibit green light emission and have low voltage, efficiency, and long lifetime characteristics.

The compounds 1-19-D23 (140 mg) and GH-1 (60 mg) were weighed, and mixed while being ground in a mortar to prepare a premix H1.

HT-2 (140 mg) and GH-1 (60 mg) were weighed, and mixed while being ground in a mortar to prepare a premix H2.

The 50% weight reduction temperatures ($T_{50}$) of the compounds 1-19-D23, HT-2, and GH-1 are shown in Table 6.

[Table 6]

| Compound name | 50% weight reduction temperature ($T_{50}$) (°C) |
|---|---|
| 1-19-D23 | 252 |
| HT-2 | 251 |
| GH-1 | 260 |

Example 26

Each thin film was laminated on the glass substrate on which an anode made of ITO having a film thickness of 110 nm was formed by a vacuum deposition method at a degree of vacuum of $4.0 \times 10^{-5}$ Pa. First, HAT-CN was formed on ITO to a thickness of 25 nm as a hole injection layer, and then HT-3 was formed to a thickness of 30 nm as a hole transport layer. Then, HT-4 was formed to a thickness of 10 nm as an electron blocking layer. Then, the premix H1 as the first host, and GD-1 as the phosphorescence dopant were co-deposited from different deposition sources to form a light emitting layer having a thickness of 40 nm. At this time, they were co-deposited under deposition conditions such that the concentration of GD-1 was 5 wt%. Then, ET-1 was formed to a thickness of 20 nm as an electron transport layer. Further, lithium fluoride (LiF) was formed on the electron transport layer to a thickness of 1 nm as an electron injection layer. Finally, aluminum (Al) was formed on the electron injection layer to a thickness of 70 nm as a cathode, whereby an organic EL device was produced.

Comparative Example 12

Each organic EL device was produced in the same manner as in Example 26, except that the second host was changed to the premix H2.

The emission color, voltage, power efficiency, and lifetime of each organic EL device produced in Examples and Comparative Examples are shown in Table 7. The emission color, voltage, and power efficiency are values at a current density of 20 mA/$cm^2$ and were initial characteristics. The time taken for the luminance to reduce to 95% of the initial luminance when the current density was 20 mA/$cm^2$ was measured as the lifetime.

[Table 7]

| | Emission color | Voltage (V) | Power efficiency (lm/W) | Lifetime (h) |
|---|---|---|---|---|
| Example 26 | Green | 4.9 | 36.5 | 107 |
| Comparative Example 12 | Green | 5.0 | 36.3 | 70 |

It is found from Examples and Comparative Examples of Table 7 that organic EL devices using the material for an organic electroluminescent device of the present invention as the host of the organic EL device containing a phosphorescence material in the light emitting layer exhibit green light emission and have low voltage, efficiency, and long lifetime characteristics.

Reference Signs List

1 substrate, 2 anode, 3 hole injection layer, 4 hole transport layer, 5 light emitting layer, 6 electron transport layer, 7 cathode.

Industrial Applicability

According to the present invention, a practically useful organic EL device having a low driving voltage, high emission efficiency, and long lifetime characteristics can be obtained.

## Claims

1. A material for an organic electroluminescent device represented by the following general formula (1):

[C1]

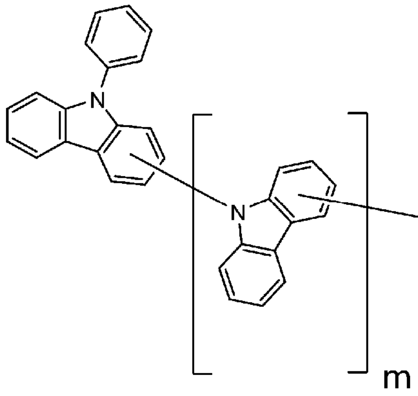

(1)

wherein m is the number of repetitions and represents an integer of 0 to 2; and a deuteration ratio in the compound represented by the general formula (1) is 50% or more.

**2.** The material for an organic electroluminescent device according to claim 1, wherein the deuteration ratio is 80% or more.

**3.** The material for an organic electroluminescent device according to claim 1 or 2, wherein the compound represented by the general formula (1) comprises at least one bonding structure represented by the following formula (2) or formula (3):

[C2]

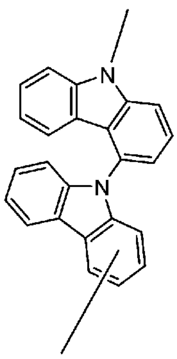

(2)

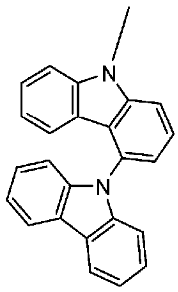

(3)

wherein some or all hydrogens in the bonding structure represented by the formula (2) and formula (3) are optionally replaced by deuteriums.

**4.** A mixed composition for an organic electroluminescent device wherein the material for an organic electroluminescent device represented by the general formula (1) according to claim 1 or 2 and a cyclic azine compound are mixed.

**5.** The mixed composition for an organic electroluminescent device according to claim 4, wherein the cyclic azine compound is represented by any one of the following general formulas (4) to (10):
[C3]

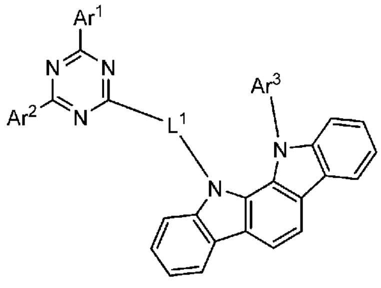
(4)

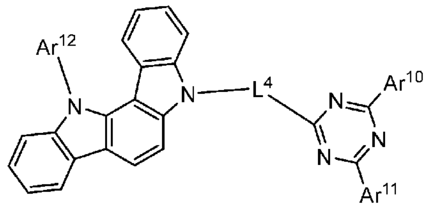
(5)

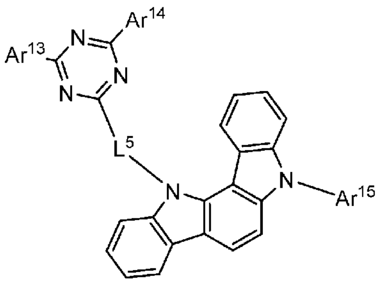
(6)

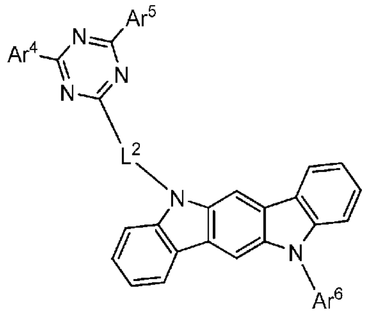
(7)

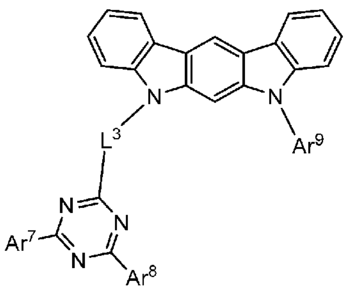
(8)

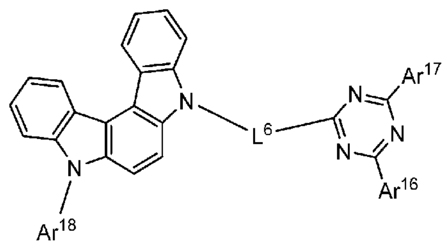
(9)

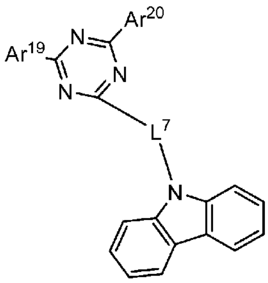
(10)

wherein $Ar^1$ to $Ar^{18}$ each independently represent a substituted or unsubstituted aromatic hydrocarbon group having 6 to 20 carbon atoms, a substituted or unsubstituted aromatic heterocyclic group having 2 to 20 carbon atoms, or a substituted or unsubstituted linked aromatic group formed by linking 2 to 3 aromatic groups selected from the group consisting of the aromatic hydrocarbon group and the aromatic heterocyclic group; $Ar^{19}$ and $Ar^{20}$ each independently represent a substituted or unsubstituted aromatic hydrocarbon group having 6 to 20 carbon atoms, a substituted or unsubstituted aromatic heterocyclic group having 2 to 17 carbon atoms, or a substituted or unsubstituted linked aromatic group formed by linking 2 to 3 aromatic groups selected from the group consisting of the aromatic hydrocarbon group and the aromatic heterocyclic group; $L^1$ to $L^7$ each independently represent a single bond or a substituted or unsubstituted phenyl group; and hydrogens in the compounds represented by the general formulas (4) to (10) are optionally replaced by deuteriums.

**6.** The mixed composition for an organic electroluminescent device according to claim 5, wherein the cyclic azine compound is represented by any one of the general formulas (4) to (6), and (10).

**7.** The mixed composition for an organic electroluminescent device according to claim 5, wherein $L^1$ to $L^7$ in the general formulas (4) to (10) are single bonds.

**8.** An organic electroluminescent device comprising one or more organic layers between an anode and a cathode

opposite to each other, wherein at least one of the organic layers contains the material for an organic electroluminescent device according to claim 1 or 2, or the mixed composition for an organic electroluminescent device according to claim 4 or 5.

**9.** The organic electroluminescent device according to claim 8, wherein at least one organic layer of the organic layers is a light emitting layer, and the organic electroluminescent device contains a thermally activated delayed fluorescence material in the light emitting layer.

**10.** The organic electroluminescent device according to claim 8, wherein at least one organic layer of the organic layers is a light emitting layer, and the organic electroluminescent device contains a phosphorescence material in the light emitting layer.

**11.** The organic electroluminescent device according to any one of claims 8 to 10, wherein the light emitting layer contains the material for an organic electroluminescent device according to claim 1 or 2 as a host material, or the mixed composition for an organic electroluminescent device according to claim 4 or 5 as a mixed host material.

**12.** A method for producing an organic electroluminescent device having a plurality of organic layers between an anode and a cathode, wherein at least one of the organic layers is a light emitting layer, the method comprising producing the light emitting layer by providing the mixed composition for an organic electroluminescent device according to any one of claims 4 to 7 and depositing the mixed composition from a deposition source.

[FIG. 1]

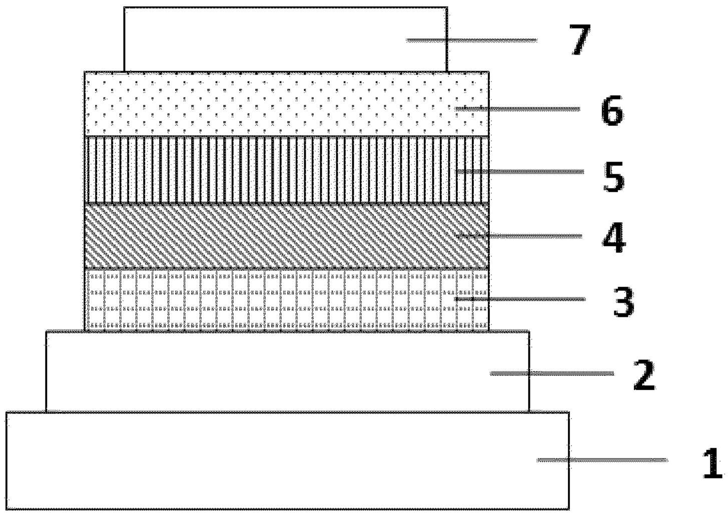

## INTERNATIONAL SEARCH REPORT

International application No.
**PCT/JP2023/004387**

**A. CLASSIFICATION OF SUBJECT MATTER**

***H10K 85/60***(2023.01)i; ***C07D 209/80***(2006.01)i; ***C09K 11/06***(2006.01)i; ***H10K 50/12***(2023.01)i; ***H10K 71/16***(2023.01)i; ***H10K 71/30***(2023.01)i; *H10K 101/10*(2023.01)n; *H10K 101/20*(2023.01)n
FI: H10K85/60; C07D209/80; C09K11/06 690; H10K50/12; H10K71/16; H10K71/30; H10K101:10; H10K101:20

According to International Patent Classification (IPC) or to both national classification and IPC

**B. FIELDS SEARCHED**

Minimum documentation searched (classification system followed by classification symbols)

H10K85/60; C07D209/80; C09K11/06; H10K50/12; H10K71/16; H10K71/30; H10K101/10; H10K101/20

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

CAplus/REGISTRY (STN)

**C. DOCUMENTS CONSIDERED TO BE RELEVANT**

| Category* | Citation of document, with indication, where appropriate, of the relevant passages | Relevant to claim No. |
|---|---|---|
| Y | WO 2016/158191 A1 (NIPPON STEEL & SUMIKIN CHEMICAL CO., LTD.) 06 October 2016 (2016-10-06)<br>paragraphs [0023], [0055]-[0059], [0091], [0094], [0099], [0110]-[0145] | 1-12 |
| Y | KR 10-2021-0092513 A (LG CHEM, LTD.) 26 July 2021 (2021-07-26)<br>paragraphs [0027], [0175] | 1-12 |
| Y | JP 2022-504349 A (ROHM AND HAAS ELECTRONIC MATERIALS KOREA LTD) 13 January 2022 (2022-01-13)<br>paragraphs [0008], [0056]-[0066] | 1-12 |
| Y | JP 2016-178326 A (E. I. DU PONT DE NEMOURS AND COMPANY) 06 October 2016 (2016-10-06)<br>claims, paragraph [0140] | 1-12 |
| Y | JP 2009-231516 A (FUJIFILM CORP) 08 October 2009 (2009-10-08)<br>paragraphs [0079], [0249] | 1-12 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **01 March 2023** | **20 March 2023** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | Telephone No. |

Form PCT/ISA/210 (second sheet) (January 2015)

**INTERNATIONAL SEARCH REPORT**

International application No.
**PCT/JP2023/004387**

**C. DOCUMENTS CONSIDERED TO BE RELEVANT**

| Category* | Citation of document, with indication, where appropriate, of the relevant passages | Relevant to claim No. |
|---|---|---|
| A | CN 108948008 A (AAC TECHNOLOGIES (NANJING) INC.) 07 December 2018 (2018-12-07) paragraph [0021] | 1-12 |
| A | CN 114075204 A (SHAANXI LTMS OPTOELECTRONICS MATERIAL CO., LTD.) 22 February 2022 (2022-02-22) claims | 1-12 |
| A | US 9312499 B1 (UNIVERSAL DISPLAY CORPORATION) 12 April 2016 (2016-04-12) entire text | 1-12 |
| P, A | US 2022/0112163 A1 (SAMSUNG DISPLAY CO., LTD.) 14 April 2022 (2022-04-14) entire text | 1-12 |
| P, A | CN 114195700 A (SHAANXI LETTER OPTOELECTRONIC MATERIAL CO., LTD.) 18 March 2022 (2022-03-18) entire text | 1-12 |
| P, A | JP 2022-132157 A (UNIVERSAL DISPLAY CORPORATION) 07 September 2022 (2022-09-07) entire text | 1-12 |

Form PCT/ISA/210 (second sheet) (January 2015)

**INTERNATIONAL SEARCH REPORT**
**Information on patent family members**

International application No.
**PCT/JP2023/004387**

| Patent document cited in search report | Publication date (day/month/year) | Patent family member(s) | Publication date (day/month/year) |
|---|---|---|---|
| WO 2016/158191 A1 | 06 October 2016 | US 2018/0083201 A1<br>paragraphs [0043], [0077], [0114], [0117], [0121], [0135]-[0171]<br>EP 3279961 A1<br>CN 107431140 A<br>KR 10-2017-0137036 A<br>TW 201704209 A | |
| KR 10-2021-0092513 A | 26 July 2021 | (Family: none) | |
| JP 2022-504349 A | 13 January 2022 | US 2021/0395215 A1<br>paragraphs [0008], [0063]-[0073]<br>WO 2020/085845 A1<br>KR 10-2020-0047398 A<br>CN 112955523 A | |
| JP 2016-178326 A | 06 October 2016 | US 2011/0037057 A1<br>claims, paragraph [0231]<br>WO 2010/075421 A2<br>EP 2401341 B1<br>TW 201035281 A<br>KR 10-2011-0106397 A<br>CN 102341475 A | |
| JP 2009-231516 A | 08 October 2009 | US 2010/0084967 A1<br>paragraphs [0066], [0251]<br>WO 2008/117889 A1<br>EP 2129739 B1<br>CN 101646745 A<br>KR 10-2010-0014603 A | |
| CN 108948008 A | 07 December 2018 | (Family: none) | |
| CN 114075204 A | 22 February 2022 | (Family: none) | |
| US 9312499 B1 | 12 April 2016 | (Family: none) | |
| US 2022/0112163 A1 | 14 April 2022 | KR 10-2022-0049622 A | |
| CN 114195700 A | 18 March 2022 | (Family: none) | |
| JP 2022-132157 A | 07 September 2022 | US 2022/0177492 A1<br>whole document<br>EP 4059915 A2<br>CN 114957298 A<br>KR 10-2022-0122557 A | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2010134350 A
- WO 2011070963 A
- WO 2012077520 A
- WO 2016158191 A
- WO 2018173593 A
- WO 2021200252 A
- US 20220020939 A
- US 20220029106 A
- JP 2013530515 A
- JP 2018002722 A
- WO 2015102118 A

### Non-patent literature cited in the description

- *J. Am. Chem. Soc.*, 2001, vol. 123, 4304
- *Adv. Mater.*, 2014, vol. 26, 7116
- *Nature*, 2012, vol. 492, 234
- *Nature Photonics*, 2014, vol. 8, 326